# EUROPEAN PATENT APPLICATION

(11) **EP 1 118 323 A2**
(43) Date of publication of application: **25.07.2001**
(21) Application number: 01300159.9
(22) Date of filing: 09.01.2001
(51) Int. Cl.: A61K 31/138, A61K 31/453, A61K 31/404, A61K 31/55, A61K 31/4453, A61K 31/40, A61K 31/4439, A61K 31/4725, A61P 43/00, A61P 9/00, A61P 35/00, A61P 19/10

(54) **Method of reducing morbidity and the risk of mortality**

(30) Priority: 12.01.2000 US 175663 P
(71) Applicant: Pfizer Products Inc., Groton, Connecticut 06340 (US)
(72) Inventor: Day, Wesley Warren, Pfizer Global Res. & Developm., Groton, Connecticut 06340 (US); Lee, Andrew George, Pfizer Global Res. & Developm., Groton, Connecticut 06340 (US); Thompson, David Duane, Pfizer Global Res.&Develop., Groton, Connecticut 06340 (US)
(74) Representative: Ruddock, Keith Stephen

(57) **Abstract**

This invention relates to methods, pharmaceutical compositions and kits useful in reducing cardiovascular morbidity and the risk of mortality in men and post-menopausal women and morbidity and the risk of mortality in post-menopausal women from the combined reduction of breast cancer, osteoporosis and cardiovascular disease by the administration of estrogen agonists / antagonists. The compositions are comprised of an estrogen agonist / antagonist and a pharmaceutically acceptable vehicle, carrier or diluent. The compositions and methods of treatment are effective while substantially reducing the concomitant liability of adverse effects associated with estrogen administration.

## Description

### FIELD OF THE INVENTION

This invention relates to compositions and methods for reducing morbidity and/or the risk of mortality. The compositions and methods utilize estrogen agonist / antagonist compounds. In both male and female subjects, cardiovascular morbitity and the risk of mortality is reduced through the administration of the estrogen agonists / antagonists of the invention. In post-menopausal women, morbidity and the risk of mortality are reduced through a combined effect of reductions in cardiovascular disease, breast cancer and osteoporosis.

### BACKGROUND OF THE INVENTION

The incidence of cardiovascular disease differs significantly between men and women, in part because of differences in risk factors and hormones (Barrett-Connor, E., Circulation 1997;95:252-64). The incidence of atherosclerotic diseases is low in premenopausal women, rises in post-menopausal women, and is reduced to premenopausal levels in post-menopausal women who receive estrogen therapy. (Stampfer, M.J., et al., N Engl J Med 1991;325:756-62.; Grady, D., et al., Ann Intern Med 1992;117:1016-37) Until recently, the atheroprotective effects of estrogen were attributed principally to the hormone's effects on serum lipid concentrations. However, estrogen-induced alterations in serum lipids account for only approximately one third of the observed clinical benefits of estrogen (Mendelsohn, M.E., Karas RH. Curr Opin Cardiol 1994;9:619-26; Bush, T.L., et al., Circulation 1987;75:1102-9). It is now believed that the direct actions of estrogen on blood vessels contribute substantially to the cardiovascular protective effects of estrogen (Farhat, M.Y., et al., FASEB J 1996;10:615-24).

The hormone estrogen has a profound effect in the vascular system of both male and female subjects although its administration is associated with other effects that can be undesirable. Estrogen increases vasodilatation and inhibits the response of blood vessels to injury and the development of atherosclerosis. Estrogen-induced vasodilatation occurs 5 to 20 minutes after estrogen has been administered and is not dependent on changes in gene expression; this action of estrogen is sometimes referred to as "nongenomic." The estrogen-induced inhibition of the response to vascular injury and the preventive effect of estrogen against atherosclerosis occur over a period of hours or days after estrogen treatment and are dependent on changes in gene expression in the vascular tissues; these actions are sometimes referred to as "genomic."

There are two estrogen receptors, estrogen receptor α and estrogen receptor β, both of which are members of the superfamily of steroid hormone receptors. (Walter, P., et al., Proc Nad Acad Sci USA 1985;82:7889-93; Kuiper, G.G.J.M., et al; Proc Nad Acad Sci USA 1996;93:5925-30) Estrogen receptors α and β have considerable homology and, like all steroid hormone receptors, are transcription factors that alter gene expression when they are activated. (Walter, P., et al. Proc Nad Acad Sci USA 1985;82:7889-93; Kuiper, G.G.J.M., et al.; Proc Nad Acad Sci USA 1996;93:5925-30; Shibata, H., et al. Recent Prog Horm Res 1997;52:141-65; Evans, R.M., Science 1988;240:889-95; Brown, M., Hematol Oncol Clin North Am 1994;8:101-12). Blood vessels are complex structures, with walls containing smooth-muscle cells and an endothelial cell lining. Vascular endothelial and smooth muscle cells bind estrogen with high affinity (Mendelsohn, M.E., et al., Curr Opin Cardiol 1994;9:619-26; Farhat, M.Y., et al., FASEB J 1996;10:615-24) and estrogen receptor α has been identified in both types of vascular cells in women and men, (Karas, R.H., et al., Circulation 1994;89:1943-50; Losordo, D.W., et al., Circulation 1994;89:1501-10; Venkov, C.D., et al., Circulation 1996;94:727-33; Kim-Schulze, S., et al., Circulation 1996;94:1402-7; Caulin-Glaser, T., et al., J Clin Invest 1996;98:36-42) as well as in myocardial cells (Grohe, C., et al., FEBS Lett 1997;416:107-12).

Estrogen receptor α activates specific target genes in vascular smooth-muscle and endothelial cells (Table 1) (Karas, R.H., et al., Circulation 1994;89:1943-50, Venkov, C.D., et al., Circulation 1996;94:727-33; Kim-Schulze, S., et al., Circulation 1996;94:1402-7; Caulin-Glaser, T., et al., J Clin Invest 1996;98:36-42; Koike, H., et al., J Vasc Surg 1996;23:477-82). Estrogen receptor β is structurally and functionally distinct from estrogen receptor α. Functional estrogen receptor (3 is also present in myocardial cells, in which it regulates the expression of nitric oxide synthases.

In premenopausal women, 17β-estradiol produced by the ovaries is the chief circulating estrogen. Serum estradiol concentrations are low in preadolescent girls and increase at menarche. In women, they range from about 100 pg per milliliter (367 pmol per liter) in the follicular phase to about 600 pg per milliliter (2200 pmol per liter) at the time of ovulation. They may rise to nearly 20,000 pg per milliliter (70,000 pmol per liter) during pregnancy. After menopause, serum estradiol concentrations fall to values similar to or lower than those in men of similar age (5 to 20 pg per milliliter [18 to 74 pmol per liter]) (Yen, S.S.C. and Jaffe, R.B., eds. Reproductive Endocrinology: Physiology, Pathophysiology and Clinical Management, 3rd ed. Philadelphia: W.B. Saunders, 1991).

Estrogen alters serum lipid concentrations, coagulation and fibrinolytic systems, antioxidant systems, and the production of other vasoactive molecules, such as nitric oxide and prostaglandins, all of which can influence the development of vascular disease.

The effects of estrogen therapy on serum lipid concentrations result largely from estrogen-receptor-mediated effects on the hepatic expression of apoprotein genes (Table 1). Many studies, including one large, randomized, controlled trial (The Writing Group for the PEPI Trial, JAMA 1995;273:199-208. [Erratum, JAMA 1995;274:1676.]) have documented that estrogen therapy in post-menopausal women decreases serum total cholesterol and low density lipoprotein (LDL) cholesterol concentrations, increases serum high-density lipoprotein (HDL) cholesterol and triglyceride concentrations, and decreases serum Lp(a) lipoprotein concentrations. Hepatic expression of the genes for several coagulation and fibrinolytic proteins is also regulated by estrogen through estrogen receptors (Table 1).

**TABLE 1.**

| ESTROGEN-REGULATED GENES OF POTENTIAL IMPORTANCE IN VASCULAR PHYSIOLOGY AND DISEASE. (Source: Mendelsohn, M.E. and Karas, R.H., N Engl J Med, 1999;340:1801-11) | |
|---|---|
| **GENE PRODUCT** | **PHYSIOLOGIC OR PATHO- PHYSIOLOGIC ROLE** |
| **Candidate estrogen-regulated genes** | |
| **(vascular cells)** | |
| | |
| Prostacyclin svnthase | Vasodilatation |
| Endothelial nitric oxide synthase | Vasodilatation |
| Inducible nitric oxide svnthase | Vasodilatation in response to vascular injury |
| Endothelin-1 | Vasoconstriction |
| Collagen | Vascular-matrix formation |
| Matrix metalloproteinase 2 | Vascular-matrix remodeling |
| E-selectin | Cell adhesion |
| Vascular-cell adhesion molecule | Cell adhesion |
| Vascular endothelial growth factor | Angiogenesis and endothelial-cell proliferation |
| | |

| **Candidate estrogen-regulated genes** | |
|---|---|
| **(nonvascular cells)** | |
| | |
| Growth- and development-related genes | |
| Transforming growth factor ß₁ | Wound healing |
| Epidermal growth factor receptor | Cell growth in response to vascular injury |
| Platelet-derived growth factor | Cell growth in response to vascular injury |
| flt-4 tyrosine kinase | Angiogenesis and endothelial-cell proliferation |
| Coagulation- and fibrinolysis -related genes | |
| Tissue factor | Hemostasis in response to thrombosis |
| Fibrinogen | Hemostasis in response to thrombosis |
| Protein S | Hemostasis in response to thrombosis |
| Coagulation factor VII | Hemostasis in response to thrombosis |
| Coagulation factor XII | Hemostasis in response to thrombosis |
| Plasminogen-activator inhibitor 1 | Hemostasis in response to thrombosis |
| Tissue plasminogen activator | Fibrinolysis |
| Antithrombin III | Anticoagulation |
| Signaling-related and miscellaneous genes | |
| Estrogen receptor α | Hormonal regulation and gene expression |
| Estrogen receptor β | Hormonal regulation and gene expression |
| Monocyte chemotactic protein 1 | Monocyte recruitment and atherosclerosis |
| I and HK2 (cardiac potassium channels) | Cardiac conduction |
| Connexin 43 | Cardiac conduction |
| Leptin | Fat metabolism and obesity |
| Apolipoproteins A, B, D, and E and Lp(a) | Lipid metabolism and atherosclerosis |
| Angiotensin-converting enzyme | Vasoconstriction |
| Angiotensin II receptor, type 1 | Vasoconstriction |

Estrogen directly regulates vasomotor tone through both short-term and long-term effects on the vasculature. Long-term administration of estrogen is associated with decreased plasma concentrations of renin (Schunkert, H., et al., Circulation 1997;95:39-45), angiotensin-converting enzyme (Proudler, A., et al., Lancet 1995;346:89-90) and endothelin-1 (Ylikorkala, O., et al., J Clin Endocrinol Metab 1995;80:3384-7) and decreased vascular expression of the gene for angiotensin II receptor type 1 (Nickenig, G., et al., Circulation 1998;97:2197-201) as well as an increased ratio of nitric oxide to endothelin-1 in plasma (Best, P.J.M., et al., Ann Intem Med 1998;128:285-8). The net effect of these changes is to promote vasodilatation.

Estrogens can cause short-term vasodilatation by both endothelium-dependent and endothelium-independent pathways. These rapid effects do not appear to involve changes in gene expression. Two mechanisms for the rapid vasodilatory effects of estrogens have been explored in some depth: effects on ion-channel function and effects on nitric oxide.

At physiologic concentrations, estrogen stimulates the opening of calcium-activated potassium channels through a nitric oxide- and cyclic guanosine monophosphate- dependent pathway (White, R.E., et al., Circ Res 1995;77:936-42; Wellman, G.C., et al., Circ Res 1996;79:1024-30) thus relaxing smooth muscle and promoting vasodilatation. These rapid effects of estrogen on vascular cells could be mediated by a known estrogen receptor, perhaps located in the plasma membrane (Pappas, T.C., et al., FASEB J 1995;9:404-10) that is able to activate nitric oxide synthase rapidly in a nongenomic manner. This suggestion is consistent with the observations that estrogen-induced stimulation of nitric oxide synthase activity in endothelial cells is blocked by specific estrogen-receptor antagonists (Chen, Z., et al., J Clin Invest 1999;103:401-6; Lantin-Hermoso, R.L., Am J Physiol 1997;273:L119-L126; Caulin-Glaser, T., et al., Circ Res 1997;81:885-92) and that estrogen receptor α can directly activate endothelial nitric oxide synthase.

Estrogen rapidly causes coronary vasodilatation ex vivo (Mendelsohn, M.E., et al., Curr Opin Cardiol 1994;9:619-26; Farhat, M.Y., et al., FASEB J 1996;10:615-24) and in vivo in cholesterol-fed ovariectomized primates (Williams, J.K., et al., J Am Coll Cardiol 1992;20:452-7) and other animals (Guetta, V., et al., Circulation 1997;96:2795-801). Estrogen dilates coronary and brachial arteries in post-menopausal women (Reis, S.E., et al., Circulation 1994;89:52-60; Gilligan, D.M., et al., Circulation 1994;89:2545-51; Gilligan, D.M., et al., Circulation 1994;90:786-91; Lieberman, E.H., et al., Ann Intern Med 1994;121:936-41; Collins, P., et al., Circulation 1995;92:24-30; Guetta, V., et al., Circulation 1997;96:2795-801) and, in some studies, in men (Collins, P., et al., Circulation 1995;92:24-30; Blumenthal, R.S., et al., Am J Cardiol 1997;80:1021-4; Reis, S.E., et al., Circulation 1998;97:23-5). Sublingual administration of 17β-estradiol in post-menopausal women increases the duration of treadmill exercise before the onset of ischemia (Rosano, G.M.C., et al., Lancet 1993;342:133-6).

Estrogen increases the expression of genes for important vasodilatory enzymes such as prostacyclin synthase and nitric oxide synthase (Table 1) (Weiner, C.P., et al., Proc Natl Acad Sci USA 1994;91;5212-6; Binko, J., et al., Am J Physiol 1998;274:H853-H859). Some of the rapid effects of estrogen may therefore be due to longer-term increases in the expression of the genes for these enzymes in vascular tissues. Estrogen may also increase the bioavailability of nitric oxide in vessels by increasing the expression of the gene for the inducible form of nitric oxide synthase (Binko, J., et al., Am J Physiol 1998;274:H853-H859). Long-term administration of estrogen increases acetylcholine-mediated coronary vasodilatation in nonhuman primates (Williams, J.K., et al., Circulation 1990;81:1680-7; Williams, J.K., et al., Circulation 1997;96:1970-5), male-to-female transsexuals (McCrohon, J.A., et al., J Am Coll Cardiol 1997;29:1432-6; New, G., et al., J Am Coll Cardiol 1997;29:1437-44), post-menopausal women (Herrington, D.M., et al., Am J Cardiol 1994;73: 951-2) and post-menopausal women with angina and normal coronary arteries (Roque, M., et al., J Am Coll Cardiol 1998;31:139-43).

Estrogen accelerates endothelial cell growth in vitro and in vivo (Morales, D.E., et al., Circulation 1995;91:755-63; Krasinski, K., et al., Circulation 1997;95:1768-72). The rapid reendothelialization induced by estrogen after vascular injury may be due in part to increased local expression of vascular endothelial growth factor. Estrogen also inhibits apoptosis of cultured human endothelial cells in an estrogen receptor-dependent manner (Spyridopoulos, I., et al., Circulation 1997;95:1505-14). Early restoration of endothelial integrity by estrogen may contribute to the attenuation of the response to injury by increasing the availability of nitric oxide, which can directly inhibit the proliferation of smooth-muscle cells (Cornwell, T.L., et al., Am J Physiol 1994;267:C14O5-C1413). Estrogen directly inhibits the migration and proliferation of smooth-muscle cells in vitro (Kolodgic, F.D., et al., Am J Pathol 1996;148: 969-76; Bhalla, R.C., et al., Am J Physiol 1997;272:H1996-H2003).

Thus estrogen has both rapid and longer-term effects on the blood-vessel wall. It is believed that estrogen influences the bioavailability of endothelial-derived nitric oxide and, through nitric oxide-mediated increases in cyclic guanosine monophosphate, causes the relaxation of vascular smooth-muscle cells. The longer-term effects of estrogen are due at least in part to changes in vascular-cell gene and protein expression that is mediated by estrogen receptor α, β, or both. Estrogen-regulated proteins influence vascular function in an autocrine or paracrine fashion.

The direct effects of estrogen on the vasculature promote vasodilatation and inhibit the development and progression of atherosclerosis. However, some of the nonvascular effects of estrogen may offset its beneficial vascular effects.

Breast cancer is a hormone-dependent disease. Women without functioning ovaries who never receive estrogen replacement do not develop breast cancer. The female-to-male ratio for the disease is about 150 to 1. A host of findings indicate that hormones play a critical role as promoters of the disease. For most epithelial malignancies, a log-log plot of incidence versus age shows a straight-line increase with every year of life. A similar plot for breast cancer shows the same straight line increase, but with a decrease in slope beginning at the age of menopause. The three dates in a woman's life that have a major impact on breast cancer incidence are age of menarche, age at first full-term pregnancy, and age of menopause. Women who experience menarche at age 16 have only 50 to 60 percent of the lifetime breast cancer risk of women who experience menarche at age 12. Similarly, menopause occurring 10 years before the median age (52 years), whether natural or surgically induced, reduces lifetime breast cancer risk by about 35 percent. Compared with nulliparous women, women who have a first full-term pregnancy by age 18 have 30 to 40 percent the risk of breast cancer. Thus, length of menstrual life--particularly the fraction occurring before the first full-term pregnancy--is a substantial component of the total risk of breast cancer. This factor can account for 70 to 80 percent of the variation in breast cancer frequency in different countries.

International variation has provided some of the most important clues on hormonal carcinogenesis. A woman living to age 80 in North America has 1 chance in 9 of developing invasive breast cancer. Asian women have one-fifth to one-tenth the risk of breast cancer of women in North America or Western Europe. Asian women have substantially lower concentrations of estrogens and progesterone. These differences cannot be explained on a genetic basis, because Asian women living in a Western environment have a risk identical to that of their Western counterparts. These women also differ markedly in height and weight from Asian women in Asia; height and weight are critical regulators of age of menarche and have substantial effects on plasma concentrations of estrogens. (Lippman, M.E., *Breast Cancer,* Chapter 91, in Harrison's Principles of Internal Medicine, 14th ed., 1998).

Menopause occurs naturally at an average age of 50 to 51 years in the USA. As ovaries age, response to pituitary gonadotropins (follicle-stimulating hormone [FSH] and luteinizing hormone [LH]) decreases, initially resulting in shorter follicular phases (thus, shorter menstrual cycles), fewer ovulations, decreased progesterone production, and more irregularity in cycles. Eventually, the follicle fails to respond and does not produce estrogen. The transitional phase, during which a woman passes out of the reproductive stage, begins before menopause. It is termed the climacteric or perimenopause, although many persons refer to it as menopause.

Premature menopause refers to ovarian failure of unknown cause that occurs before age 40. It may be associated with smoking, living at high altitude, or poor nutritional status. Artificial menopause may result from oophorectomy, chemotherapy, radiation of the pelvis, or any process that impairs ovarian blood supply.

Symptoms of the climacteric range from nonexistent to severe. Hot flushes (flashes) and sweating secondary to vasomotor instability affect 75% of women. Most have hot flushes for more than 1 year, and 25 to 50% for more than 5 years. The woman feels warm or hot and may perspire, sometimes profusely. The skin, especially of the head and neck, becomes red and warm. The flush, which may last from 30 sec to 5 min, may be followed by chills. Vasomotor symptoms of the hot flush coincide with the onset of LH pulses, but not every increase in LH is associated with a hot flush, suggesting that hypothalamic control of LH pulses is independent of that of flushes. This independence is confirmed by the occurrence of hot flushes in women who have had pituitary failure and do not secrete LH and/or FSH.

Psychologic and emotional symptoms--including fatigue, irritability, insomnia, inability to concentrate, depression, memory loss, headache, anxiety, and nervousness and timidity can occur. Sleep disruption by recurrent hot flushes contributes to fatigue and irritability. Intermittent dizziness, paresthesias, palpitations, and tachycardia may also occur. Nausea, constipation, diarrhea, arthralgia, myalgia, cold hands and feet, and weight gain are also common.

The large reduction in estrogen leads to profound changes in the lower genital tract; e.g., the vaginal mucosa and vulvar skin become thinner, the normal bacterial flora changes, and the labia minora, clitoris, uterus, and ovaries decrease in size. Inflammation of the vaginal mucosa (atrophic vaginitis) can cause the mucosa to have a strawberry appearance and can lead to urinary frequency and urgency, vaginal dryness, and dyspareunia. Women tend to lose pelvic muscle tone and to develop urinary incontinence, cystitis, and vaginitis.

The incidence of clinical coronary artery disease (CAD) in premenopausal women is very low. However, following menopause, the atherogenic risk factors increase (e.g., increased LDL, reduced HDL) and the rate of clinical coronary events accelerates to the levels observed in men. Diabetes mellitus, which is more common in women, greatly increases the occurrence of clinical CAD and amplifies the deleterious effects of hypertension, hyperlipidemia, and smoking. The post-menopausal use of hormone replacement therapy (estrogen with or without a progestin) reduces adverse coronary outcomes but should not be given to patients at higher than usual risk of breast cancer.

Over and above obstetric and gynecologic conditions, women experience or report greater morbidity than men. Studies have found that women experience a 25 percent higher rate of restricted activity and a 40 percent higher rate of bed disability, adjusted across all ages. For example, 22.2 percent of women aged 85 and older require assistance with activities of daily living, compared to 14.5 percent of men in this age group. Women also make more visits to physicians, particularly for acute self-limited illnesses. It is unclear whether there are real differences in the prevalence of morbidity or differences in care-seeking behavior following the perception of symptoms.

In the developed nations, women live longer than men. In 1995, in the United States, the projected average life expectancy from birth is 79.7 years for females and 72.8 years for males. Although more male fetuses are conceived than female fetuses, females have a survival advantage over males in all age groups. The longer life expectancy of women in developed countries, compared to men, is due in large part to the difference in mortality caused by ischemic heart disease (IHD).

As shown in Table 2, the leading causes of death among young women in the United States are accidents, homicide, and suicide. During the middle years, breast cancer is a slightly more common cause of death than IHD and lung cancer. In women between ages 65 and 74, IHD, lung cancer, and cerebrovascular disease overtake breast cancer as the leading causes of death. Among women of all ages, IHD is the leading cause of death, by a substantial margin, with a mortality rate five to sixfold higher than the rate for either lung or breast cancer.

**Table 2:**

| Death Rates (Per 100,000) For The Leading Causes Of Death In U.S. Women, 1990 | | | |
|---|---|---|---|
| Ages 24-34 | Ages 45-54 | Ages 65-74 | All Ages |
| Total: 74.2 | Total: 342.7 | Total 1991.2 | Total 812.0 |
| 1. Motor vehicle accidents (11.5) | 1. Breast cancer (45.4) | 1. Ischemic heart disease (415.2) | 1. Ischemic heart disease (185.6) |
| 2. Homicide (7.2) disease (33.6) (181.7) disease(68.6) | 2. Ischemic heart | 2. Lung cancer^{*} | 2. Cerebrovascular |
| 3. Suicide (5.6) | 3. Lung cancer (35.3)^{*} | 3. Cerebrovascular disease (126.9) | 3. Lung cancer^{*} (40.4) |
| 4. Non-motor vehicle accidents | 4. Cerebrovascular disease (17.0) | 4. Breast cancer (111.7) | 4. Breast cancer (34.0) |
| SOURCE: Adapted from National Center for Health Statistics Vital Statistics of the United States, 1990, vol II: Mortality, Part A. Washington, Public Health Service, 1994. DHHS Publication No. (PHS)95-1101, pp 40-52. | | | |

| | | | |
|---|---|---|---|
| ^{*} Cancer of respiratory and intrathoracic organs, predominantly lung cancer. | | | |

Certain biological differences also contribute to the greater longevity of women. The most obvious is the exposure to estrogen that most women experience for about 40 years of their lives, which may provide some protection from IHD.

Primary prevention and screening are crucial elements in improving the health of women. Although there are few definitive clinical trials of preventive interventions in women, a growing number of large case-control and prospective cohort observational studies (such as the Nurses' Health Study) have evaluated the prevention of disease in women. Based on available literature and experience, various authoritative organizations (e.g., the U.S. Preventive Services Task Force, Canadian Task Force, American College of Physicians, American Heart Association, American Cancer Society) have published guidelines on preventive practices in women (Blumenthal, S.J. et al: Towards a Women's Health Research Agenda. Findings of the Scientific Advisory Meeting. Washington, DC, Bass and Howes, 1991; Colditz, G.A, JAMWA 50:40, 1995; Gijsbers van Wijk, C.M.T., et al., Women Health 17:91, 1991; Verbrugge, L.M. and Wingard, D.L., Women Health 12:103, 1987).

Post-menopausal estrogen therapy is associated with a 40 to 50 percent reduction in deaths due to IHD, a finding supported by several case-control studies. Calcium and estrogen replacement therapy have been shown to slow the development of osteoporosis and to reduce the frequency of hip and vertebral fracture in the post-menopausal woman (Rich-Edwards, J.W., et al., N Engl J Med 332:1758, 1995).

Many people think of IHD as primarily a problem of men, perhaps because men have more than twice the total incidence of cardiovascular morbidity and mortality than women between the ages of 35 and 84. However, as stated earlier, in the United States IHD is the leading cause of death among women as well as men (Table 1), although the curve for mortality rate from IHD lags behind that for men by about a decade. Nearly 250,000 women die annually from IHD ; after age 40, one in three women will die from heart disease. Although IHD mortality has been falling in the United States over the past 30 years, the rate of decline has been lower in women than in men.

Women have a more favorable risk profile for IHD in some respects: higher HDL cholesterol levels, lower triglyceride levels, and less upper-body obesity than men. But women also have a less favorable risk profile in other respects: more obesity, higher blood pressure, higher plasma cholesterol levels, higher fibrinogen levels, and more diabetes. An explanation for the sex differential in IHD is a cardioprotective effect of estrogen, due to improvement of the lipid profile, a direct vasodilatory effect, and perhaps other factors. HDL cholesterol levels appear to be a particularly important risk factor for IHD in women. HDL levels are higher in all age groups in women compared to men, and are higher in premenopausal and estrogen-treated post-menopausal women than in non-estrogen-treated post-menopausal women.

IHD presents differently in men and women. In the Framingham study, angina was the most frequent initial symptom of IHD in women, occurring in 47 percent of women. Myocardial infarction was the most frequent initial symptom in men, occurring in 46 percent of men. The exercise electrocardiogram appears to have a lower specificity for IHD in women than in men.

Hypertension is more common in U.S. women than men, largely owing to the high prevalence of hypertension in older age groups and the longer survival of women. Renovascular hypertension from fibromuscular dysplasia occurs more often in women. Other causes of secondary hypertension occur with equal frequency in women and men. Both the effectiveness and the adverse effects of various antihypertensive drugs appear to be comparable in women and men. Benefits of treatment for severe hypertension have been dramatic in both women and men. However, in clinical trials of the treatment of mild to moderate hypertension, women have had a smaller decrease in morbidity and mortality than men, perhaps because women have a lower risk of myocardial infarction and stroke than men to begin with. Older women benefit at least as much as men from treatment, as demonstrated by the Systolic Hypertension in the Elderly study. When oral contraceptives were first introduced, many women had a small increase in blood pressure, with 5 percent showing an increase to above 140/90 over a 5-year period. The incidence of hypertension appears to be lower with the current low-dose oral contraceptives. Post-menopausal estrogen therapy is not associated with increases in blood pressure (Kaplan, N.M., Arch Intern Med 185:563, 1995).

Estrogen secretion also appears to play an important role in the control of bone density. While it is clear that the risk of osteoporosis in post-menopausal women is much greater than in men of the same age, and while post-menopausal estrogen supplementation is associated with a decreased incidence of osteoporosis, the mechanisms by which estrogen exerts its protective effects are not fully elucidated.

The Framingham Study, an observational study of men and women under way since 1947, has been increasingly analyzing data specific to women. Since 1976, the Nurses' Health Study has been following more than 120,000 women, prospectively collecting data about their smoking, diet, physical activity, medications, prevention and screening behaviors, and some psychosocial factors. This study already has revealed information about how these factors contribute to the relative risk of developing a number of medical disorders, including breast cancer, ischemic heart disease, stroke, diabetes, and fracture, as well as to causes of mortality (Murabito, J.M., JAMWA 50:35, 1995).

The Post-menopausal Estrogens/Progestins Intervention (PEPI) Trial was the first major trial testing the effects of post-menopausal hormone replacement therapy. It was a 3-year, multicenter, randomized, double-blind, placebo-control trial of the effects of three estrogen/progestin regimens on risk factors for cardiovascular disease, bone mineral density, and endometrial tissue. Thus far, the study has reported that estrogen alone or in combination with progestin increased serum levels of HDL and decreased low-density lipoprotein (LDL) and fibrinogen levels. While unopposed estrogen (without progestins) resulted in the most beneficial effects on lipids, it also was associated with an increased risk of endometrial hyperplasia (The Writing Group for the PEPI Trial, JAMA 273:199, 1995).

The compositions and methods of the present invention act to cure, ameliorate, or prevent pathological conditions that contribute to cardiovascular mortality in men and post-menopausal women and reduce morbidity and the risk of mortality in post-menopausal women due to the combined reduction of cardiovascular disease, breast cancer and osteoporosis. These effects are accomplished by the compositions and methods of the invention with a substantial reduction of the concomitant liability of adverse effects associated with estrogen administration. Not being bound by any single theory, it is believed that administration of the compositions of the invention results in an estrogenic effect on the cardiovascular system and bone while having an antiestrogenic effect on the breast and other tissues. The overall effect is a beneficial one that is substantially free of the adverse effects attributed to estrogen administration.

### BRIEF DESCRIPTION OF THE DRAWINGS

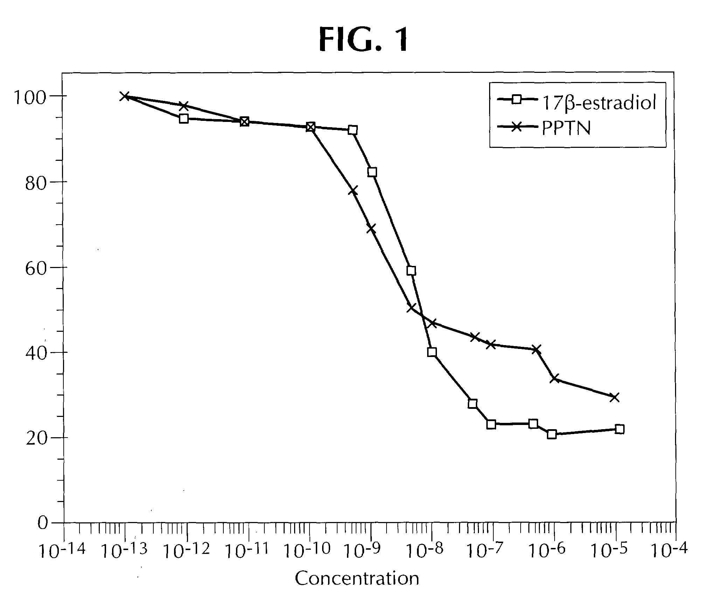

Figure 1 is a log-linear competition binding plot of PPTN and 17β-estradiol to human estrogen receptor. The X-axis represents percentage of radiolabeled estrogen bound to receptor. The Y-axis represents the molar concentration of added ligand. Values are mean ± SEM.

### SUMMARY OF THE INVENTION

This invention relates to pharmaceutical compositions useful for the reduction of morbidity and the risk of mortality. The compositions are comprised of an estrogen agonist / antagonist and a pharmaceutically acceptable carrier, vehicle or diluent. These compositions are effective in reducing cardiovascular morbidity and the risk of mortality in male and female subjects and for reducing morbidity and/or the risk of mortality in post-menopausal women due to the combined effect of reductions in cardiovascular disease, breast cancer and osteoporosis.

A second aspect of the invention relates to methods of reducing morbidity and mortality. Specifically the methods relate to methods of reducing cardiovascular morbidity and the risk of mortality in male and female subjects and reducing morbidity and/or the risk of mortality in post-menopausal women due to the combined effect of reductions of cardiovascular disease, breast cancer and osteoporosis. The methods comprise the administration of an effective amount of the estrogen agonists / antagonists as described herein.

A third aspect of the invention is that the compositions and methods of reducing cardiovascular morbidity and the risk of mortality in male and female subjects and reducing morbidity and/or the risk of mortality due to the combined effect of reductions of cardiovascular disease, breast cancer and osteoporosis are effective while substantially reducing the concomitant liability of adverse effects associated with estrogen administration.

As a fourth aspect, the present invention provides for kits for use by a consumer to reduce cardiovascular morbidity and the risk of in male and female subjects and reduce morbidity and/or the risk of mortality due to the combined effect of reductions of cardiovascular disease, breast cancer and osteoporosis. The kit comprises a) a pharmaceutical composition comprising an estrogen agonist / antagonist of the present invention and a pharmaceutically acceptable carrier, vehicle or diluent; and b) instructions describing a method of using the pharmaceutical composition to reduce cardiovascular mortality in men and post-menopausal women and reduce morbidity and the risk of mortality due to the combined effect of reductions of cardiovascular disease, breast cancer and osteoporosis. The instructions may also indicate that the kit is for the reduction of cardiovascular mortality in men and post-menopausal women and/or the reduction of morbidity and/or the risk of mortality due to the combined effects of cardiovascular disease, breast cancer and osteoporosis, while substantially reducing the concomitant liability of adverse effects associated with estrogen administration.

As a fifth aspect, the present invention provides for the use of estrogen agonists / antagonists of the present invention for the manufacture of a medicament to reduce cardiovascular in male and female subjects and reduce morbidity and/or the risk of mortality due to the combined effect of reductions of cardiovascular disease, breast cancer and osteoporosis in post menopausal women. These indications are also treated by the medicament while substantially reducing the concomitant liability of adverse effects associated with estrogen administration.

### DETAILED DESCRIPTION OF THE INVENTION

The present invention relates to compositions and methods for the reduction of morbidity and for the reduction of the risk of mortality. Unless otherwise specified, the following terms have the meanings as defined below:

"Treatment" as used herein includes preventative (e.g., prophylactic) and palliative treatment and "treating" as used herein refers to the act of providing preventative and/or palliative treatment.

A "subject" is an animal including the human species that is treatable with the compositions, methods and kits of the present invention. The term "subject" or "subjects" is intended to refer to both the male and female gender unless one gender is specifically indicated.

"Adverse effects associated with estrogen" include breast tenderness, breast cancer, bloating, headache, increased blood clotting and menstrual bleeding in women. Unopposed estrogen therapy increases the risk of endometrial carcinoma. Women on long-term estrogen therapy may have an increased risk that is not reversed by concurrent progestin (N Engl J Med 332:1589, 1995). In men, the adverse effects of estrogen include increased blood clotting, gynecomastia, feminization and decreased libido.

The term "post-menopausal women" is defined to include not only women of advanced age who have passed through menopause, but also women who have been hysterectomized or for some other reason have suppressed estrogen production, such as those who have undergone long-term administration of corticosteroids, suffer from Cushions' syndrome or have gonadal dysgenesis.

"Breast cancer" is defined as a malignant proliferation of epithelial cells lining the ducts or lobules of the breast.

An "estrogen agonist / antagonist" is compound that affects some of the same receptors that estrogen does, but not necessarily all, and in some instances, it antagonises or blocks estrogen. It is also known as a "selective estrogen receptor modulator" (SERM). Estrogen agonist / antagonists may also be referred to as antiestrogens although they have some estrogenic activity at some estrogen receptors. Estrogen agonist / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 22:707, 1973, Capony, et al., Mol Cell Endocrinol, 3:233, 1975).

The method referred to above for reducing morbidity and/or mortality generally refers to benefits and/or survival in the long term. Clinical benefits may be observable within a few weeks, for example 2-3 weeks, however, this does not imply that the patients are not benefiting from the treatment prior to actual clinical observation. It is preferred, however that administration be effected long term; that is for longer than 16 weeks, and preferably longer than 6 months.

Not being bound by any single theory, it is believed that the estrogen agonists / antagonists of the present invention and the compositions containing those estrogen agonists / antagonists reduce the risk of cardiovascular mortality in men and reduce morbidity and the risk of mortality in post-menopausal women due to activity at the estrogen receptor. The estrogen agonists / antagonists of the present invention exert an estrogenic cardioprotective effect in animals and an estrogenic effect on bone to treat osteoporosis. The estrogen agonists / antagonists of the present invention exert an anti-estrogenic effect on breast tissue to prevent or treat breast cancer in animals. The effects are achieved without the concomitant liability of adverse effects associated with estrogen administration due to the estrogen agonists / antagonists antiestrogen effects in other tissues such as breast tissue.

The estrogen agonist / antagonists of the present invention include the compounds described in US 5,552,412 which is incorporated in its entirety. The compounds are described by formula (I) given below: 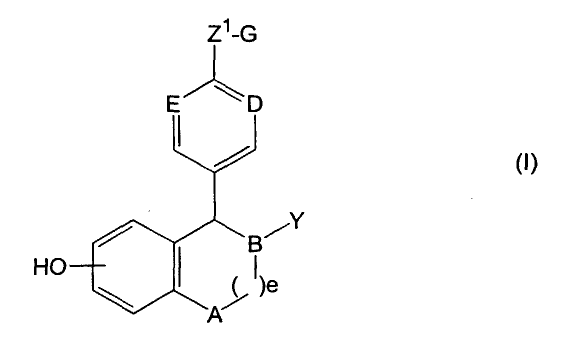 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚ W(CH₂)_{q}-;
   (b) -O(CH₂)ₚ CR⁵R⁶-;
   (c) -O(CH₂)ₚW(CH₂)_{q}-;
   (d) -OCHR²CHR³-; or
   (e) -SCHR²CHR³-;
   G is
      (a) -NR⁷R⁸;
      (b) 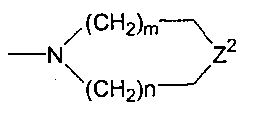 wherein n is 0,1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
      (c) a bicyclic amine containing five to twelve carbon atoms, either bridged or fused and optionally substituted with 1-3 substituents independently selected from R⁴; or
   Z¹ and G in combination may be 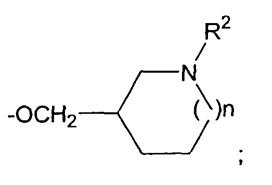
   W is
      (a) -CH₂-;
      (b) -CH=CH-;
      (c) -O-;
      (d) -NR²-;
      (e) -S(O)ₙ-;
      (f) 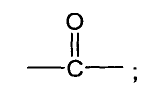
      (g) -CR²(OH)-;
      (h) -CONR²-;
      (i) -NR²CO-;
      (j) 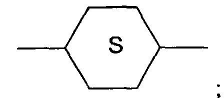 or
      (k) -C≡C-;
   R is hydrogen or C₁-C₆ alkyl;
   R² and R³ are independently
      (a) hydrogen; or
      (b) C₁-C₄ alkyl;
   R⁴ is
      (a) hydrogen;
      (b) halogen;
      (c) C₁-C₆ alkyl;
      (d) C₁-C₄ alkoxy;
      (e) C₁-C₄ acyloxy;
      (f) C₁-C₄ alkylthio;
      (g) C₁-C₄ alkylsulfinyl;
      (h) C₁-C₄ alkylsulfonyl;
      (i) hydroxy (C₁-C₄)alkyl;
      (j) aryl (C₁-C₄)alkyl;
      (k) -CO₂H;
      (l) -CN;
      (m) -CONHOR;
      (n) -SO₂NHR;
      (o) -NH₂;
      (p) C₁-C₄ alkylamino;
      (q) C₁-C₄ dialkylamino;
      (r) -NHSO₂R;
      (s) -NO₂;
      (t) -aryl; or
      (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
   a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
and optical and geometric isomers thereof; and nontoxic
pharmacologically acceptable acid addition salts, N-oxides, esters, quaternary ammonium salts and prodrugs thereof.

By halo is meant chloro, bromo, iodo, or fluoro or by halogen is meant chlorine, bromine, iodine or fluorine.

By alkyl is meant straight chain or branched saturated hydrocarbon. Exemplary of such alkyl groups (assuming the designated length encompasses the particular example) are methyl, ethyl, propyl, isopropyl, butyl, sec-butyl, tertiary butyl, pentyl, isopentyl, hexyl and isohexyl.

By alkoxy is meant straight chain or branched saturated alkyl bonded through an oxy. Exemplary of such alkoxy groups (assuming the designated length encompasses the particular example) are methoxy, ethoxy, propoxy, isopropoxy, butoxy, isobutoxy, tertiary butoxy, pentoxy, isopentoxy, hexoxy and isohexoxy.

The parenthetical negative or positive sign used herein in the nomenclature denotes the direction plane polarized light is rotated by the particular stereoisomer.

Additional preferred compounds of the invention are of the formula (IA): 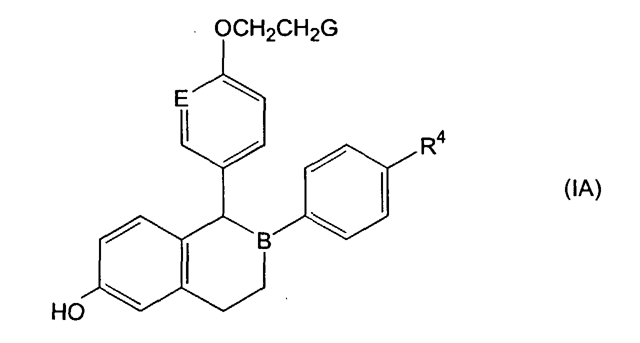 wherein G is

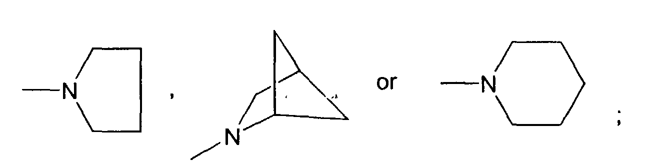

R⁴ is H, OH, F, or Cl; and B and E are independently selected from CH and N.

Especially preferred compounds of the invention for the compositions and methods are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydro-naphthalene-2-ol;
cis-6-phenyl-5-[4-(2-pyrrolidin-1-yl-ethoxy)-phenyl]-5,6,7,8-tetrahydro-naphthalene-2-ol;
cis-1-[6'-pyrrolidinoethoxy-3'-pyridyl]-2-phenyl-6-hydroxy-1,2,3,4-tetrahydronaphthalene;
1-(4'-pyrrlidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol; and
1-(4'-pyrrlidinolethoxyphenyl)-2-phenyl-6-hydroxy-1,2,3,4-tetrahydroisoquinoline and their salts. An especially preferred salt of the above is the tartrate salt.

Other preferred estrogen agonists / antagonists are described in US 5,047,431. The structure of these compounds is given by formula (II) below: 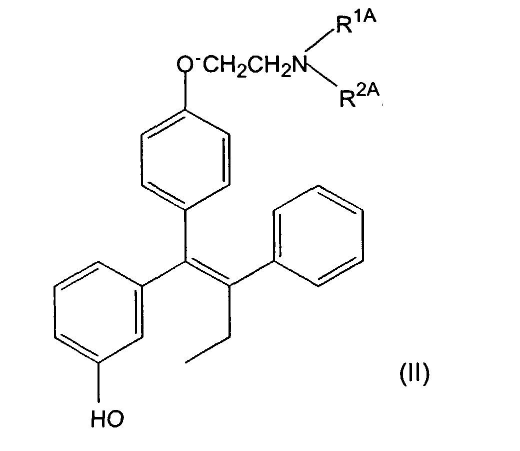 wherein
R^{1A} and R^{2A} may be the same or different provided that, when R^{1A} and R^{2A} are the same, each is a methyl or ethyl group, and, when R^{1A} and R^{2A} are different, one of them is a methyl or ethyl group and the other is hydrogen or a benzyl group; and pharmaceutically acceptable salts and prodrugs thereof.

Additional preferred estrogen agonists / antagonists are tamoxifen: (ethanamine, 2-[-4-(1,2-diphenyl-1-butenyl)phenoxy]-N,N-dimethyl, (Z)-2-, 2-hydroxy-1,2,3-propanetricarboxylate(1:1)) and other compounds as disclosed in U.S. Patent 4,536,516; 4-hydroxy tamoxifen (i.e., tamoxifen wherein the 2-phenyl moiety has a hydroxy group at the 4 position) and other compounds as disclosed in U.S. Patent 4,623,660; raloxifene: (methanone, [6-hydroxy-2-(4-hydroxyphenyl)benzo[b]thien-3-yl][4-[2-(1-piperidinyl)ethoxy]phenyl]-,hydrochloride) and other compounds as disclosed in U.S. Patents 4,418,068, 5,393,763, 5,457,117, 5,478,847 and 5,641,790; toremifene: (ethanamine, 2-[4-(4-chloro-1,2-diphenyl-1-butenyl)phenoxy]-N,N-dimethyl-, (Z)-, 2-hydroxy-1,2,3-propanetricarboxylate (1:1) and other compounds as disclosed in U.S. Patents 4,696,949 and 4,996,225; centchroman: 1-[2-[[4-(-methoxy-2,2, dimethyl-3-phenyl-chroman-4-yl)-phenoxy]-ethyl]-pyrrolidine and other compounds as disclosed in U.S. Patent 3,822,287; idoxifene: pyrrolidine, 1-[-[4-[(1-(4-iodophenyl)-2-phenyl-1-butenyl]phenoxy]ethyl] and other compounds as disclosed in U.S. Patent 4,839,155; 6-(4-hydroxy-phenyl)-5-[4-(2-piperidin-1-yl-ethoxy)-benzyl]-naphthalen-2-ol and other compounds as disclosed in U.S. Patent 5,484,795; and {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone and other compounds as disclosed in published international application WO 95/10513. Other preferred compounds include GW 5638 and GW 7604. The synthesis of these compounds is described in Willson et al., J. Med. Chem., 1994;37:1550-1552.

Further preferred estrogen agonists / antagonists include EM-652 (as shown in the formula designated herein as formula (III) and EM-800 (as shown in the formula designated herein as formula (IV)). The synthesis of EM-652 and EM-800 and the activity of various enantiomers is described in Gauthier et al., J. Med.Chem., 1997;40:2117-2122.

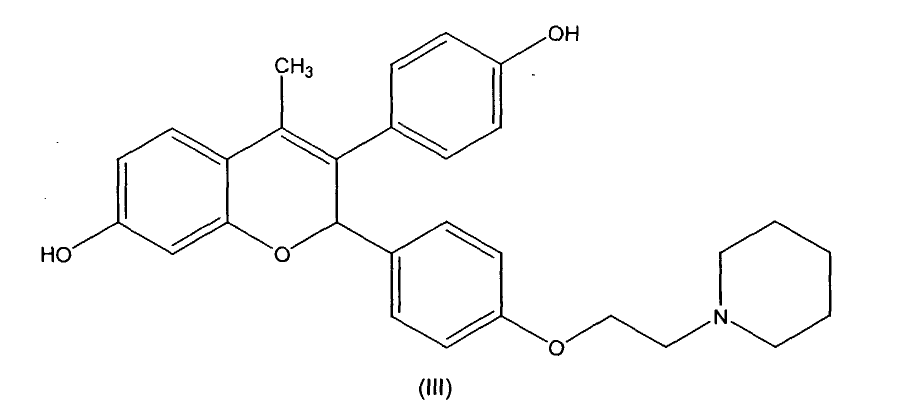

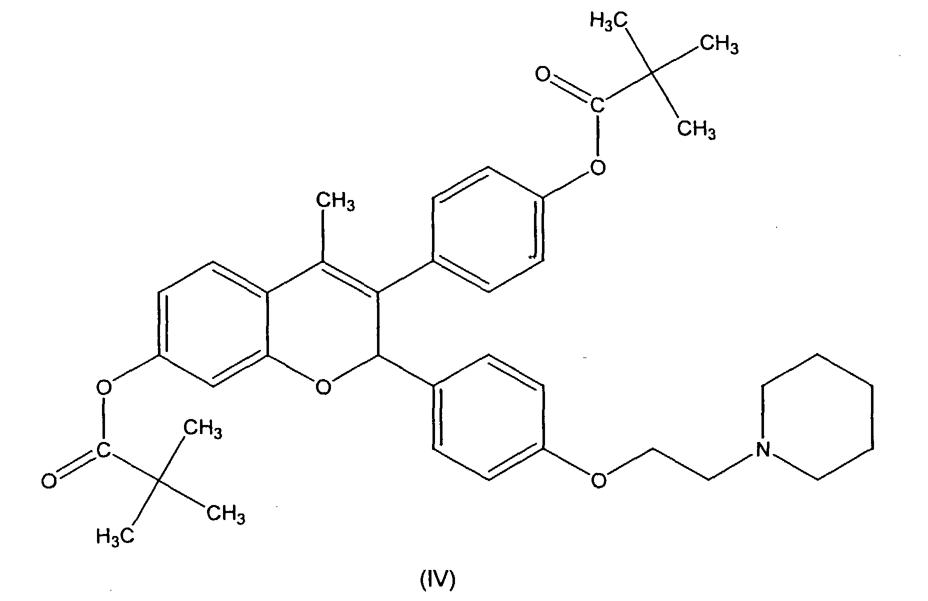

Further preferred estrogen agonists / antagonists include TSE 424 and other compounds disclosed in U.S. Patent 5,998,402, U.S. Patent 5,985,910, U.S. Patent 5,780,497, U.S. Patent 5,880,137, and European Patent Application EP 0802183 A1 including the compounds described by the formulae designated herein as formulae V and VI, below: 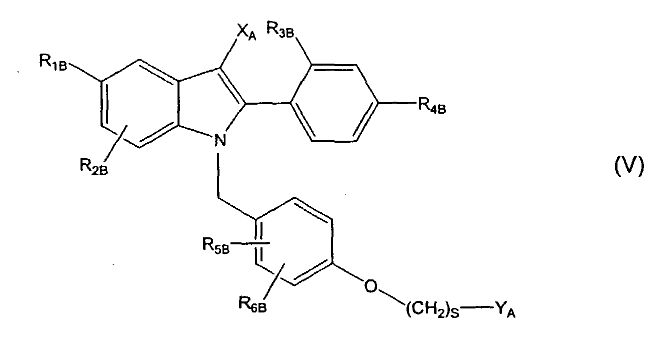 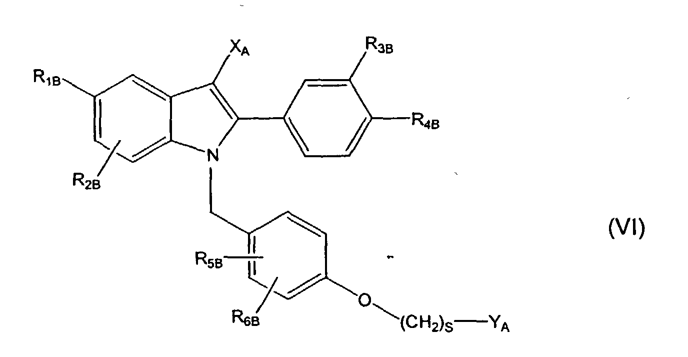 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including trifluoromethyl ether and trichloromethyl ether.
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including trifluoromethyl ether and trichloromethyl ether, cyano, C₁-C₆ alkyl (straight chain or branched), or trifluoromethyl;
X_{A} is selected from H, C₁-C₆ alkyl, cyano, nitro, trifluoromethyl, and halogen;
s is 2 or 3;
Y_{A} is selected from:
   a) the moiety: 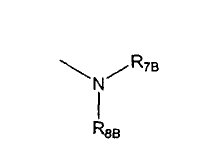 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
   b) a five-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR₁, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
   e) a bicyclic heterocycle containing from 6-12 carbon atoms either bridged or fused and containing up to two heteroatoms selected from the group consisting of -O-, -NH-, -N(C₁-C₄ alkyl)-, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H-, -CN-, -CONHR_{1B}-, -NH₂, -N=, C₁-C₄ alkylamino, di(C₁-C₄)alkylamino, -NHSO₂R_{1B}, -NHCOR_{1B}, -N02, and phenyl optionally substituted with 1-3 (C₁-C₄) alkyl; and optical and geometric isomers thereof; and nontoxic pharmacologically acceptable acid addition salts, N-oxides, esters, quaternary ammonium salts, and prodrugs thereof.

The more preferred compounds of this invention are those having the general structures V or VI, above, wherein:
R_{1B} is selected from H, OH or the C₁-C₁₂ esters or alkyl ethers thereof, and halogen;
R_{2B}, R_{3B}, R_{4B}, R_{5B}, and R_{6B} are independently selected from H, OH or the C₁-C₁₂ esters or alkyl ethers thereof, halogen, cyano, C₁-C₆ alkyl, or trihalomethyl, preferably trifluoromethyl, with the proviso that, when R_{1B} is H, R_{2B} is not OH;
X_{A} is selected from H, C₁-C₆ alkyl, cyano, nitro, trifluoromethyl, and halogen;
Y_{A} is the moiety: 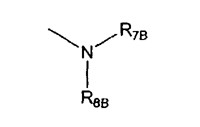
R_{7B} and R_{8B} are selected independently from H, C₁-C₆ alkyl, or combined by -(CH₂)_{w}-, wherein w is an integer of from 2 to 6, so as to form a ring, the ring being optionally substituted by up to three substituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, - CO₂H, -CN, -CONH(C₁-C₄alkyl), -NH₂, C₁-C₄ alkylamino, C₁-C₄ dialkylamino, -NHSO₂(C₁-C₄alkyl), -CO(C₁-C₄alkyl), and -NO₂; and optical and geometric isomers thereof; and nontoxic pharmacologically acceptable acid addition salts, N-oxides, esters, quaternary ammonium salts, and prodrugs thereof.

The rings formed by a concatenated R_{7B} and R_{8B}, mentioned above, may include, but are not limited to, aziridine, azetidine, pyrrolidine, piperidine, hexamethyleneamine or heptamethyleneamine rings.

The most preferred compounds of structural formulas V and VI, above, are those wherein R_{1B} is OH; R_{2B} - R_{6B} are as defined above; X_{A} is selected from the group of Cl, NO₂, CN, CF₃, or CH₃; Y_{A} is the moiety 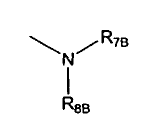 and R_{7B} and R_{8B} are concatenated together as -(CH₂)ₜ-, wherein t is an integer of from 4 to 6, to form a ring optionally substituted by up to three subsituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄)alkyl, -NH2, C₁-C₄ alkylamino, di(C₁-C₄)alkylamino, -NHSO₂(C₁-C₄)alkyl, -NHCO(C₁-C₄)alkyl, and -NO₂; and optical and geometric isomers thereof; and nontoxic pharmacologically acceptable acid addition salts, N-oxides, esters, quaternary ammonium salts, and prodrugs thereof.

Another preferred compound is TSE-424 as described by the formula designated herein as formula (Va) below:

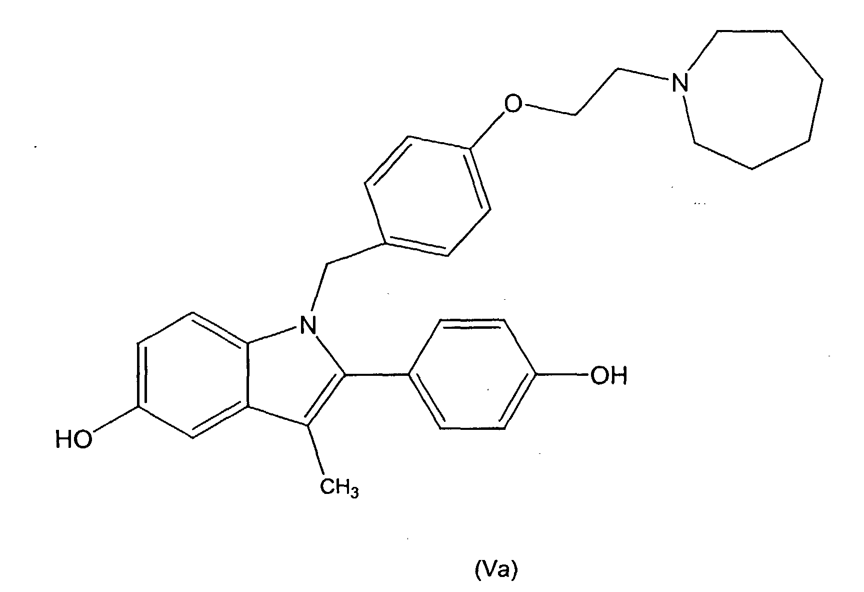

The chemist of ordinary skill will recognize that certain compounds of this invention will contain one or more atoms which may be in a particular stereochemical, tautomeric, or geometric configuration, giving rise to stereoisomers, tautomers and configurational isomers. All such isomers and mixtures thereof are included in this invention. Hydrates of the compounds of this invention are also included.

It is also part of the present invention to administer more than one estrogen agonist / antagonist. In addition, an estrogen agonist /antagonist or combinations of estrogen agonists / antagonists can be administered in combination with other therapeutically active compounds, particularly compounds that are used to treat rheumatoid arthritis, colon cancer, tissue wounds or cataracts. The different compounds can be administered in the same dosage form or in different dosage forms at the same time or at different times.

The chemist of ordinary skill will recognize that certain compounds of this invention will contain one or more atoms which may be in a particular stereochemical, tautomeric, or geometric configuration, giving rise to stereoisomers, tautomers and configurational isomers. All such isomers and mixtures thereof are included in this invention. Hydrates of the compounds of this invention are also included.

The subject invention also includes isotopically-labeled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labeled compounds of the present invention, for example those into which racioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds of Formula I of this invention and prodrugs thereof can generally be prepared by carrying out the procedures outlined and/or exemplified in U.S. patent 5,552,412 and by substituting a readily available isotopically labeled reagent for a non-isotopically labeled reagent.

Pharmaceutical chemists will easily recognize that physiologically active compounds which have accessible hydroxy groups are frequently administered in the form of pharmaceutically acceptable esters. The literature concerning such compounds, such as estradiol, provides a great number of instances of such esters. The compounds of this invention are no exception in this respect, and can be effectively administered as an ester, formed on the hydroxy groups, just as one skilled in pharmaceutical chemistry would expect. It is believed that such esters are metabolically cleaved in the body, yielding the compound with a free hydroxy group. It is possible, as has long been known in pharmaceutical chemistry, to adjust the rate or duration of action of the compound by appropriate choices of ester groups.

Certain ester groups are preferred as constituents of the compounds of this invention. The compounds of formula I or IA may contain ester groups at various positions as defined herein above, where these groups are represented as -COOR₉, R₉ is C₁ -C₁₄ alkyl, C₁ -C₃ chloroalkyl, C₁ -C₃ fluoroalkyl, C₅ -C₇ cycloalkyl, phenyl, or phenyl mono- or disubstituted with C₁ -C₄ alkyl, C₁ -C₄ alkoxy, hydroxy, nitro, chloro, fluoro or tri(chloro or fluoro)methyl.

The pharmaceutically acceptable acid addition salts of the compounds of this invention may be formed of the compound itself, or of any of its esters, and include the pharmaceutically acceptable salts which are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferable with hydrochloric acid, citric acid, benzoic acid, maleic acid, acetic acid and propionic acid.

The compounds of this invention, as discussed above, can be administered in the form of acid addition salts. The salts are conveniently formed, as is usual in organic chemistry, by reacting a compound of this invention with a suitable acid, such as have been described above. The salts are quickly formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if a compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6,7,8-tetrahydro-naphthalene-2-ol is the D-(-)-tartrate salt.

The dose of a compound of this invention to be administered to a subject is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts and other dosage amounts set forth elsewhere in this specification and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses of the free base form of the estrogen agonists / antagonists. Calculation of the dosage amount for other forms of the free base form such as salts or hydrates is easily accomplished by performing a simple ratio relative to the molecular weights of the species involved.

The general range of effective administration rates of the estrogen agonists / antagonists is from about 0.001 mg/day to about 10 mg/day. A preferred rate range is from about 0.010 mg/day to 5 mg/day. Of course, it is often practical to administer the daily dose of compound in portions, at various hours of the day. However, in any given case, the amount of compound administered will depend on such factors as the solubility of the active component, the formulation used and the route of administration.

The route of administration of the compounds of this invention is not critical. The compounds are known to be absorbed from the alimentary tract, and so it is usually preferred to administer a compound orally for reasons of convenience. However, the compounds may equally effectively be administered percutaneously, or as suppositories for absorption by the rectum, if desired in a given instance. All of the usual types of compositions may be used, including tablets, chewable tablets, capsules, solutions, parenteral solutions, troches, suppositories and suspensions. Compositions are formulated to contain a daily dose, or a convenient fraction of daily dose, in a dosage unit, which may be a single tablet or capsule or convenient volume of a liquid.

In general, all of the compositions are prepared according to methods usual in pharmaceutical chemistry and by those procedures outlined and/or exemplified in U.S. patent 5,552,412.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant is necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which swell when wetted to break up the tablet and release the compound. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavor and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Even the parenteral preparations may be made long-acting, by dissolving or suspending the compound in oily or emulsified vehicles which allow it to disperse only slowly in the serum.

The term "prodrug" means compounds that are transformed in vivo to yield a compound of the present invention. The transformation may occur by various mechanisms, such as through hydrolysis in blood. A good discussion of the use of prodrugs is provided by T. Higuchi and W. Stella, "Pro-drugs as Novel Delivery Systems," Vol. 14 of the *A.C.S. Symposium Series,* and in *Bioreversible Carriers in Drug Design,* ed. Edward B. Roche, American Pharmaceutical Association and Pergamon Press, 1987.

For example, if a compound of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C6)alkanoyloxy)ethyl, 1-methyl-1-((C₁-C₆)alkanoyloxy)ethyl, (C₁-C6)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound of the present invention comprises an amine functional group, a prodrug can be formed by the replacement of a hydrogen atom in the amine group with a group such as R^{X}-carbonyl, R^{X}O-carbonyl, NR^{X}R^{X}'-carbonyl where R^{X} and R^{X}' are each independently ((C₁-C₁₀)alkyl, (C₃-C₇)cycloalkyl, benzyl, or R^{X}-carbonyl is a natural α-aminoacyl or natural α-aminoacyl-natural α-aminoacyl, -C(OH)C(O)OY^{X} wherein (Y^{X} is H, (C₁-C₆)alkyl or benzyl), -C(OY^{X0}) Y^{X1} wherein Y^{X0} is (C₁-C₄) alkyl and Y^{X1} is ((C₁-C₆)alkyl, carboxy(C₁-C₆)alkyl, amino(C₁-C₄)alkyl or mono-N- or di-N,N-(C₁-C₆)alkylaminoalkyl, -C(Y^{X2}) Y^{X3} wherein Y^{X2} is H or methyl and Y^{X3} is mono-N- or di-N,N-(C₁-C₆)alkylamino, morpholino, piperidin-1-yl or pyrrolidin-1-yl.

As used herein, the term "effective amount" means an amount of compound of the methods of the present invention that is capable of treating the symptoms of the described pathological conditions. The specific dose of a compound administered according to this invention will, of course, be determined by the particular circumstances surrounding the case including, for example, the compound administered, the route of administration, the state of being of the patient, and the severity of the pathological condition being treated.

Advantageously, the present invention also provides kits for use by a consumer for reducing cardiovascular morbidity and the risk of in male and female subjects or morbidity and/or the risk of mortality in post-menopausal women due to the combined effects of cardiovascular disease, breast cancer and osteoporosis. The kits comprise a) a pharmaceutical composition comprising an estrogen agonist / antagonist and a pharmaceutically acceptable carrier, vehicle or diluent; and b) instructions describing a method of using the pharmaceutical composition for reducing cardiovascular morbidity and the risk of mortality in male and female subjects or morbidity and/or the risk of mortality in post-menopausal women due to the combined effects of cardiovascular disease, breast cancer and osteoporosis. The instructions may also indicate that the kit is for reducing cardiovascular morbidity and the risk of mortality in male and female subjects or morbidity and/or the risk of mortality in post-menopausal women due to the combined effects of cardiovascular disease, breast cancer and osteoporosis while substantially reducing the concomitant liability of adverse effects associated with estrogen administration.

A "kit" as used in the instant application includes a container for containing the separate compositions such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle which is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It maybe desirable to provide a written memory aid, where the written memory aid is of the type containing information and/or instructions for the physician, pharmacist or subject, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday,"... etc .... "Second Week, Monday, Tuesday, ..." etc. Other variations of memory aids will be readily apparent. A "daily dose" can be a single tablet or capsule or several tablets or capsules to be taken on a given day. When the kit contains separate compositions, a daily dose of one or more compositions of the kit can consist of one tablet or capsule while a daily dose of another one or more compositions of the kit can consist of several tablets or capsules.

Another specific embodiment of a kit is a dispenser designed to dispense the daily doses one at a time in the order of their intended use. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Based on a reading of the present description and claims, certain modifications to the compositions and methods described herein will be apparent to one of ordinary skill in the art. The claims appended hereto are intended to encompass these modifications.

All references and patents cited herein are incorporated by reference.

### EXAMPLES

### Example 1: Estrogen Receptor Binding.

### Estrogen and estrogen agonist / antagonist binding affinity was measured by the following protocol:

cDNA cloning of human ERα: The coding region of human ERα was cloned by RT-PCR from human breast cancer cell mRNA using Expand™ High Fidelity PCR System according to manufacturer's instructions (Boehringer-Mannheim, Indianapolis, IN). PCR products were cloned into pCR2.1 TA Cloning Kit (Invitrogen, Carlsbad, CA) and sequenced. Each receptor coding region was subcloned into the mammalian expression vector pcDNA3 ((Invitrogen, Carlsbad, CA).

Mammalian cell expression. Receptor proteins were overexpressed in 293T cells. These cells, derived from HEK293 cells (ATCC, Manassas, VA), have been engineered to stably express large T antigen and can therefore replicate plasmids containing a SV40 origin of replication to high copy numbers. 293T cells were transfected with either hERα-pcDNA3 or hERβ-pcDNA3 using lipofectamine as described by the manufacturer (Gibco/BRL, Bethesda, MD). Cells were harvested in phosphate buffered saline (PBS) with 0.5 mM EDTA at 48 h post-transfection. Cell pellets were washed once with PBS/EDTA. Whole cell lysates were prepared by homogenization in TEG buffer (50 mM Tris pH 7.4, 1.5 mM EDTA, 50 mM NaCI, 10% glycerol, 5 mM DTT, 5 µg/ml aprotinin, 10 µg/ml leupeptin, 0.1 mg/ml Pefabloc) using a dounce homogenizor. Extracts were centrifuged at 100,000 x g for 2 h at 4C and supernatants were collected. Total protein concentrations were determined using BioRad reagent (BioRad, Hercules, CA).

Competition binding assay. The ability of various compounds to inhibit [³H]-estradiol binding was measured by a competition binding assay using dextran-coated charcoal as has been described (Leake RE, Habib F 1987 Steroid hormone receptors: assay and characterization. In: B. Green and R.E. Leake (eds). Steroid Hormones a Practical Approach. IRL Press Ltd, Oxford. 67-92.) 293T cell extracts expressing either hERα or hERβ were incubated in the presence of increasing concentrations of competitor and a fixed concentration of [³H]-estradiol (141 µCi/mmol, New England Nuclear, Boston, MA) in 50 mM TrisHCl pH 7.4, 1.5 mM EDTA, 50 mM NaCI, 10% glycerol, 5 mM DTT, 0.5 mg/mL β-lactoglobulin in a final volume of 0.2 mL. All competitors were dissolved in dimethylsulfoxide. The final concentration of receptor was 50 pM with 0.5 nM [³H]-estradiol. After 16 h at 4C, dextran-coated charcoal (20 µL) was added. After 15 min at room temperature the charcoal was removed by centrifugation and the radioactive ligand present in the supernatant was measured by scintillation counting. All reagents were obtained from Sigma (St. Louis, MO) unless otherwise indicated.

The binding affinity of (-)-cis-6-phenyl-5-[4-(2-pyrrolidin-1-yl-ethoxy)-phenyl]-5,6,7,8-tetrahydro-naphthalene-2-ol (PPTN) and 17β-estradiol were measured using recombinant human estrogen receptor (ER). Figure 1 shows the results of a binding experiment in which the binding of PPTN was found to be similar to that of 17β-estradiol.

### Example 2: Inhibition of In Vitro Human Breast Tumor Cell Growth.

The *in vitro* antiproliferative effects of (-)-cis-6-phenyl-5-[4-(2-pyrrolidin-1-yl-ethoxy)-phenyl]-5,6,7,8-tetrahydro-naphthalene-2-ol (PPTN) were tested using two types of human breast cancer cell lines: first, MCF-7 cells, which contain ER as well as progesterone receptors (PgR), and second, MDA-MB-231 cells, which lack ER and PgR, and enable the determination of an effect that is independent of the ER mechanism. The effect of PPTN on the growth of these different cell lines was determined by incubation of the cells with various compound concentrations for 6 days.

The antiproliferative effects were then determined by direct cell counts. PPTN inhibited the growth of the ER-positive cell line MCF-7. The IC₅₀ for growth inhibition was approximately 3 to 5 x 10⁻¹¹ M. In MDA-MB-231, ER-negative cell lines, the compound did not inhibit cell proliferation. These results indicate that growth inhibition was ER-specific and not due to cytotoxicity since the compound had no observable effect on the ER-negative cell line.

### Example 3: Effect of (-)-Cis-6-phenyl-5-[4-(2-pyrrolidin-1-yl-ethoxy)-phenyl]-5,6,7,8-tetrahydro-naphthalene-2-ol (PPTN) in the Ovariectomized Rat Model: A Model of Post-menopausal Osteoporosis.

In women, estrogen deficiency during the menopause results in increased bone turnover leading to bone loss. Ovariectomy in rats produces estrogen deficiency and increased bone turnover leading to trabecular bone loss similar to that observed in post-menopausal women (Kalu, D.N., Bone and Mineral 1991;15:175; Frost, H.M., Jee W.S.S., Bone and Mineral 1992;18:227; Wronski, T.J., Yen, C-F, Cells Materials 1991;(suppl. 1):69). The OVX rat is thus an appropriate model to evaluate compounds for the prevention and treatment of post-menopausal osteoporosis. The ability of PPTN to inhibit estrogen deficiency bone loss was assessed in 5-month-old OVX rats, since ovariectomy causes significant bone loss in the lumbar vertebrae, proximal tibia, and distal femoral metaphyses (Ke, H.Z., et al., Endocrin 1995;136:2435; Chen, H.K., et al., J Bone Miner Res 1995;10:1256).

Five-month-old OVX female rats were treated with PPTN at oral doses of 0.733, 7.33, 73.3, and 733 µg/kg/day, or 17α-ethynyl estradiol (EE) at 30 µg/kg/day (10 rats per subgroup) daily for 4 weeks. The oral treatment began 1 day after surgery. Groups of vehicle-treated sham rats (n = 10) and vehicle-treated OVX rats (n = 10) served as controls. Calcein at 10 mg/kg was injected s.c. to all rats 12 and 2 days before necropsy as a fluorochrome bone marker to measure bone dynamic histomorphometric parameters. The effects of PPTN on the following end points were determined: (a) serum osteocalcin, a biochemical marker of bone turnover, (b) bone mineral density of lumbar vertebrae and distal femoral metaphyses, (c) bone histomorphometry of fifth lumbar vertebral body and proximal tibial metaphyses.

Serum osteocalcin concentration was determined by radioimmunoassay. Four weeks after surgery, serum osteocalcin concentration increased significantly by 85% in vehicle-treated OVX controls, compared with vehicle-treated sham controls. This increase was completely prevented by treatment with PPTN at doses ≥ 7.33 µg/kg/day, or by treatment with EE. Therefore, both 7.33 µg/kg/day of PPTN and EE prevented the increase in osteocalcin concentration induced by estrogen deficiency in rats. These data suggest that PPTN inhibits bone turnover induced by estrogen deficiency.

The first to the sixth lumbar vertebrae from each rat were removed during necropsy. These were then scanned ex *vivo* using dual-energy X-ray absorptiometry. The scan images were analyzed, and bone area, bone mineral content (BMC), and bone mineral density (BMD) of whole lumbar vertebrae (WLV), and LV1 through LV6 were determined. Bone areas of WLV, and LV1 through LV6 did not differ between groups. The changes in BMC were similar to those observed in BMD. Further, the changes in BMD of WLV were identical to those observed in each of six vertebrae. Therefore, only the changes in the whole lumbar vertebrae are reported.

Following ovariectomy with vehicle treatment, a significant decrease in BMD of lumbar vertebrae was found at 4 weeks after ovariectomy. PPTN at all dose levels and EE completely prevented the decrease in lumbar vertebral BMD. Furthermore, BMD in OVX rats treated with 73.3 µg/kg/day of PPTN increased significantly as compared with both sham and OVX controls. These data indicate that PPTN at doses as low as 0.733 µg/kg/day completely prevented lumbar vertebral bone loss induced by estrogen deficiency.

In order to understand the cellular mechanism of bone-protective effects of PPTN at the tissue level, bone histomorphometric methods were used to determine the effects of PPTN on cancellous bone of the fifth lumbar vertebral body in OVX rats. Ovariectomy caused significant decreases in trabecular bone volume (trabecular bone area divided by marrow space area, expressed as percent) and trabecular number, and a significant increase in trabecular separation of the fifth lumbar vertebral body. These changes induced by ovariectomy were completely prevented by treatment with all doses of PPTN and EE. Further, trabecular number was significantly increased in OVX rats treated with EE or PPTN at 73.3 or 733 µg/kg/day as compared with sham controls. These data revealed that PPTN is a bone protective agent in estrogen-deficient rats. The ED₅₀ of PPTN in preserving lumbar vertebral cancellous bone mass and structure in OVX rats was less than 0.733 µg/kg/day.

A significant increase in percent eroded surface (+59%), an index of osteoclastic bone resorption, was found in vehicle-treated OVX rats compared with vehicle-treated sham controls. Similar to EE, PPTN at all dose levels significantly decreased percent eroded surface in OVX rats compared with sham controls. Therefore, PPTN prevented bone loss in OVX rats by inhibiting bone resorption associated with estrogen deficiency.

Ovariectomy resulted in a significant increase in bone formation rate (BFR, amount of bone formation per unit bone surface), an index of bone turnover, while EE and PPTN at all dose levels inhibited this increase.

Bone turnover rate was significantly increased in vehicle-treated OVX rats compared with vehicle-treated sham controls. Bone turnover rate in OVX rats treated with either PPTN or EE did not differ from sham controls, indicating PPTN at all dose levels and EE completely prevented the increase in bone turnover associated with estrogen deficiency.

In summary, PPTN prevented lumbar vertebral bone loss by inhibiting bone resorption and bone turnover associated with estrogen deficiency in a manner indistinguishable from estrogen. The ED₅₀ of PPTN in preserving trabecular bone in this model was less than 0.733 µg/kg/day.

Using dual-energy X-ray absorptiometry, the right femur of each rat was scanned *ex vivo.* Bone mineral density (BMD) of the distal femoral metaphyses (second 0.5 cm from the distal end of femur) and the proximal femur (the first 0.5 cm from the proximal end of femur, which contains the femoral head, neck, and greater trochanter) was determined.

Four weeks after ovariectomy in vehicle-treated rats, BMD of distal femoral metaphyses and proximal femora significantly decreased. EE prevented the decrease in BMD of distal femoral metaphyses, but had no effect on proximal femoral BMD. In OVX rats treated with PPTN at 0.733 or 7.33 µg/kg/day, BMD of distal femoral metaphyses and proximal femur did not differ from OVX controls. However, PPTN at both 73.3 or 733 µg/kg/day completely prevented the decreases in BMD of distal femoral metaphyses and proximal femur in OVX rats.

In order to determine the effect of PPTN on long bone metaphyses, histomorphometric analyses were performed on the proximal tibiae. Trabecular bone volume significantly decreased in vehicle-treated OVX controls compared with sham controls. PPTN at doses ≥ 7.33 µg/kg/day had significantly greater trabecular bone volume compared with OVX controls. At 0.733 µg/kg/day, PPTN had no significant effect on trabecular bone volume. The ED₅₀ of PPTN in preserving proximal tibial trabecular bone mass in OVX rats was between 0.733 and 7.33 µg/kg/day.

Significant increases in indices of osteoclastic bone resorption (osteoclast number per millimeter of bone surface and percent osteoclast surface) were found in vehicle-treated OVX rats compared with vehicle-treated sham controls. Similar to EE, PPTN at 7.33, 73.3, or 733 µg/kg/day dose levels significantly decreased osteoclast number per millimeter of bone surface and percent osteoclast surface compared with both sham and OVX controls. Further, percent osteoclast surface decreased significantly in OVX rats treated with PPTN at 0.733 µg/kg/day compared with OVX controls.

Ovariectomy significantly increased the bone turnover rate in proximal tibial trabecular bone compared with vehicle-treated sham controls. The bone turnover rate in OVX rats treated with either PPTN or EE did not differ from sham controls with the exception of PPTN at the 0.733 µg/kg/day dose, indicating that PPTN at doses of 7.33 to 733 µg/kg/day prevented the increase in bone turnover associated with estrogen deficiency and maintained it at sham control levels.

In summary, PPTN had similar effects in preserving bone mineral density and trabecular bone volume in both proximal tibiae and lumbar vertebrae. The discrepancy between lumbar vertebrae and proximal tibiae in response to PPTN administration was found only at 0.733 µg/kg/day, which indicated that PPTN at 0.733 µg/kg/day completely prevented bone loss in lumbar vertebrae and failed to do so in proximal tibiae. Therefore, the ED₅₀ of PPTN in preserving cancellous bone mass and inhibiting bone resorption was less than 0.733 µg/kg/day in lumbar vertebrae, and between 0.733 and 7.33 µg/kg/day in proximal tibiae.

### Example 4: Nitric Oxide Formation by Cultured Endothelial Cells

NO-formation is assessed by determination of intracellular cyclic GMP in cultured endothelial cells, whereas release of NO from these cells is measured by the stimulatory effect of NO on the activity of soluble guanylyl cyclase (Lückhoff, et al, Br J Pharmacol, 95:189, 1988; Wiemer, et al, Hypertension, 18:558, 1991; Linz, et al, J Mol Cell Cardiol, 24:909, 1992).

Bovine or porcine aorta is obtained and endothelial cells are isolated by digestion with dispase. The cells are seeded on 6- or 24-well plates and grown to confluence. Dulbecco's modified Eagle's/Ham's F-12 medium containing 20% fetal calf serum is supplemented with penicillin (10 U/ml), streptomycin (10 mg/ml), L-glutamate (1 mM/l), glutathione (5 mg/ml), and L(+)ascorbic acid (5 mg/ml).

Primary cultures of endothelial cells are used. After removal of the culture medium by aspiration, the monolayer is washed twice with 2 ml HEPES-Tyrode's solution (37°C). Thereafter, the cells are preincubated for 15 min at 37°C with 3-isobutyl-1-methyl-xanthine (IBMX), (10⁻⁴ M). After this time, compounds or solvents are added. After predetermined periods, the incubation medium is quickly removed. The cells are then immediately extracted with 0.6 ml 6% trichloroacetic acid and scraped off with a rubber scraper. The cell suspension is sonicated for 10 sec before being centrifuged for 5 min at 4,000g. The supernatants are extracted with four volumes of water saturated diethylether, and the samples frozen (-20°C) until analysis. The protein contents of the samples are measured according to Lowry, et al (J Biol Chem, 193:265, 1951). Cyclic GMP can be determined in the acetylated samples by various methods (Heath et al, Which Cyclic GMP Assay?, in Moncada, S., et al., (eds) The Biology of Nitric Oxide: 2 Enzymology, Biochemistry and Immunology. Portland Press, London, pp 98, 1992), e.g., using a commercially available radio-immunoassay (New England Nuclear). Cyclic GMP content is expressed as picomoles GMP per milligram protein.

Release of NO from endothelial cells is assayed on the basis of the stimulatory effect of NO on the activity of soluble guanylyl cyclase (purified from bovine lung) (Gerzer, et al, Eur J Biochem 116:479, 1981). The activity of the enzyme is determined in terms of the formation of cyclic [³²P]GMP from α-[³²P]GTP. Reactions are carried out in a reaction mixture containing 30 mM triethanolamine HCI (pH 7.4), 1 mM reduced glutathione, 4 mM MgCl₂, 1 mM cGMP and 0.1 mg/ml bovine g-globulin (total volume of 0.18 ml) at 37°C in the presence of α-[³²P]GTP (0.03 mM; 0.2 mCi) and soluble guanylyl cyclase (4 mg). Ten ml samples are quickly transferred to the reaction mixture. Enzymatic formation of cGMP is allowed to proceed for 60 sec and then stopped by the addition of 450 ml zinc acetate (120 mM) and 500 ml sodium carbonate (120 mM). A complete inhibition of cGMP formation can be achieved by preincubation of the monolayers for 30 min with the stereospecific inhibitor of NO synthase, *N*^{G}-nitro-L-arginine.

Time-response curves and dose-response curves are obtained after addition of the estrogen agonists / antagonists of the present invention. Data are reported as mean values ± SEM of cGMP (pmol / mg protein) or guanylyl cyclase activity (nmol / mg / min).

### Example 5: Morbidity and Risk of Mortality in Stable Cardiovascular Disease.

Effects of estrogen agonists / antagonists for reducing cardiovascular morbidity and/or the risk of mortality is assessed in an at risk population utilizing electrocardiogram (ECG) measurements as a surrogate endpoint. The presence of myocardial ischemia, as evidenced by the ECG, is an independent, noninvasive predictor of adverse clinical outcomes in patients with stable coronary disease (CAD). The presence of ST-segment depression on the resting ECG predicts increased morbidity and mortality (Chaitman, B.R., et al., J Am Coll Cardiol 1995;26:585; Stone, P.H., et al., Am J Cardiol 1997;80:1395). The development of exercise-induced ST-segment depression is the standard noninvasive marker of poor prognosis and serves as the fundamental clinical guide to identify the need for coronary angiography and revascularization of patients with CAD. Episodes of ST-segment depression during routine daily activities, identified by ambulatory ECG (Holter) monitoring, provides incremental prognostic information. In a double-blind randomized clinical study, at risk patients receive either an estrogen agonist / antagonist or a placebo. ECG is monitored continuously via Holter monitoring or through daily or regular ECG measurements. A significant elimination or reduction of ST-segment depression in the patients receiving estrogen agonist / antagonist indicates a likely reduction of cardiovascular morbidity and/or the risk of mortality.

## Claims

1. The use of estrogen agonists/antagonists of formula (I): 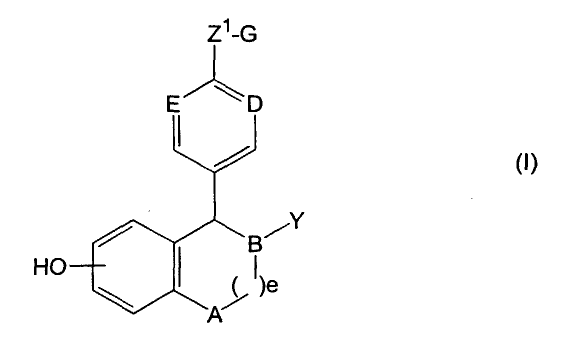 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ Cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚ W(CH₂)_{q}-;
(b) -O(CH₂)ₚ CR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 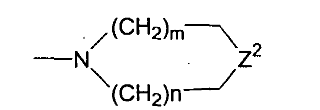 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 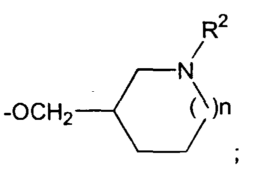 W is
(a) -CH₂-;
(b) -CH=CH-;
(c) -O-;
(d) -NR²-;
(e) -S(O)ₙ-;
(f) 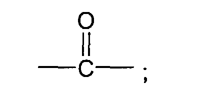
(g) -CR²(OH)-;
(h) -CONR²-;
(i) -NR²CO-;
(j) 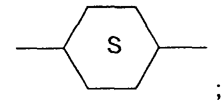 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(l) -CN;
(m) -CONHOR;
(n) -SO₂NHR;
(o) -NH2;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a nontoxic pharmacologically acceptable acid addition salt, N-oxide, ester, quaternary ammonium salt, or a prodrug thereof, in the manufacture of a medicament to reduce cardiovascular mortality in male and female subjects and reduce morbidity and/or the risk of mortality due to the combined effect of reductions of cardiovascular disease, breast cancer and osteoporosis in post menopausal women.

2. The use as claimed in claims 1 wherein the estrogen agonist/antagonist is a compound of formula (IA): 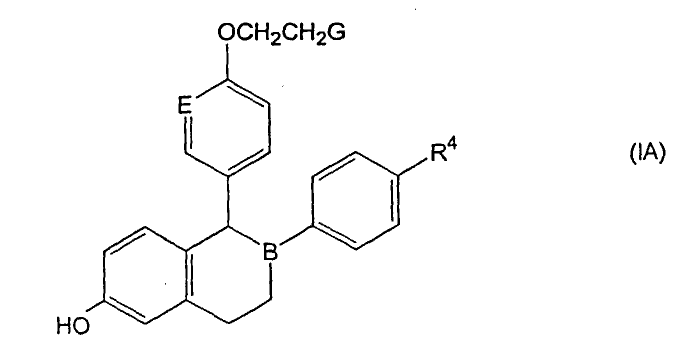 wherein G is 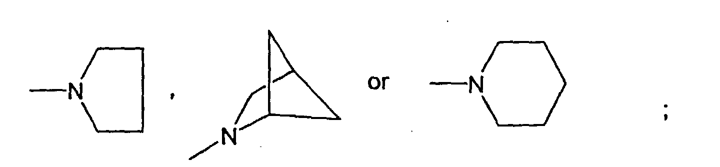 R⁴ is H, OH, F, or Cl; and B and E are independently selected from CH and N or an optical or geometric isomer thereof; or a nontoxic pharmacologically acceptable acid addition salt, N-oxide, ester, quaternary ammonium salt, or a prodrug thereof.

3. The use as claimed in claim 1 wherein the estrogen agonist / antagonist is a member of the group consisting of:
cis-6-(4-fluoro-phenyl)-5-[4-(2-piperidin-1-yl-ethoxy)-phenyl]-5,6,7,8-tetrahydro-naphthalene-2-ol,
(-)-cis-6-phenyl-5-[4-(2-pyrrolidin-1-yl-ethoxy)-phenyl]-5,6,7,8-tetrahydro-naphthalene-2-ol,
cis-6-phenyl-5-[4-(2-pyrrolidin-1-yl-ethoxy)-phenyl]-5,6,7,8-tetrahydro-naphthalene-2-ol,
cis-1-[6'-pyrrolidinoethoxy-3'-pyridyl]-2-phenyl-6-hydroxy-1,2,3,4-tetrahydronaphthalene,
1-(4'-pyrrlidinoethoxyphenyl)-2-(4"-fluorophenyl)-6-hydroxy-1,2,3,4-tetrahydroisoquinoline,
cis-6-(4-hydroxyphenyl)-5-[4-(2-piperidin-1-yl-ethoxy)-phenyl]-5,6,7,8-tetrahydro-naphthalene-2-ol, and
1-(4'-pyrrlidinolethoxyphenyl)-2-phenyl-6-hydroxy-1,2,3,4-tetrahydroisoquinoline and optical or geometric isomers thereof; and nontoxic pharmacologically acceptable acid addition salts, N-oxides, esters, quaternary ammonium salts, and prodrugs thereof.

4. The use as claimed in claim 1 wherein the compound is cis-6-(4-fluoro phenyl)-5-[4-(2-piperidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

5. The use as claimed in claim 1 wherein the compound is (-)-cis-6-phenyl-5-[4-(2-pyrrolidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

6. The use as claimed in claim 1 wherein the compound is cis-6-phenyl-5-[4-2-pyrrolidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

7. The use as claimed in claim 1 wherein the compound is cis-1-[6'-pyrrolidinoethoxy-3'-pyridyl]-2-phenyl-6-hydroxy-1,2,3,4-tetrahydronaphthalene or an optical or geometric isomer thereof; or a nontoxic pharmacologically acceptable acid addition salt, N-oxide, ester, quaternary ammonium salt, or a prodrug thereof.

8. The use as claimed in claim 1 wherein the compound is 1-(4'-pyrrlidinoethoxyphenyl)-2-(4"-fluorophenyl)-6-hydroxy-1,2,3,4-tetrahydroisoquinoline or an optical or geometric isomer thereof; or a nontoxic pharmacologically acceptable acid addition salt, N-oxide, ester, quaternary ammonium salt, or a prodrug thereof.

9. The use as claimed in claim 1 wherein the compound is cis-6-(4-hydroxyphenyl)-5-[4-(2-piperidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

10. The use as claimed in claim 1 wherein the compound is 1-(4'-pyrrlidinolethoxyphenyl)-2-phenyl-6-hydroxy-1,2,3,4-tetrahydroisoquinoline or an optical or geometric isomer thereof; or a nontoxic pharmacologically acceptable acid addition salt, N-oxide, ester, quatemary ammonium salt, or a prodrug thereof.

11. The use a claimed in claim 5 wherein the estrogen agonist / antagonist is in the form of a D-tartrate salt.

12. The use as claimed in claim 1 wherein the medicament is for the reduction of morbidity and/or the risk of cardiovascular mortality.

13. The use as claimed in claim 1 wherein the medicament is for the reduction of morbidity and/or the risk of mortality in post-menopausal women through the combined effects of reductions in cardiovascular disease, breast cancer and osteoporosis.

14. The kit comprising:
a) a pharmaceutical composition comprising a compound of formula (I): 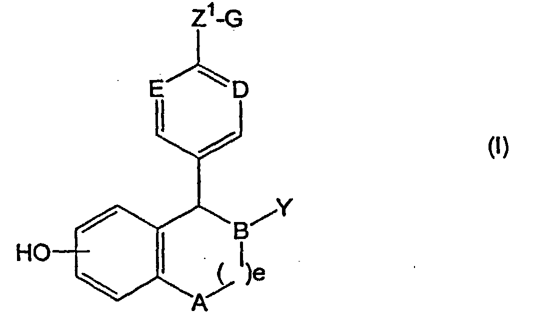 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚ W(CH₂)_{q}-;
(b) -O(CH₂)ₚ CR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 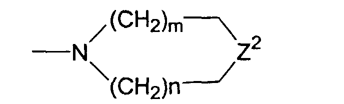 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴: or
Z¹ and G in combination may be 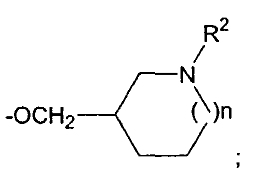
W is
(a) -CH₂-;
(b) -CH=CH-;
(c) -O-;
(d) -NR²-;
(e) -S(O)ₙ-;
(f) 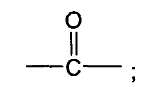
(g) -CR²(OH)-;
(h) -CONR²-;
(i) -NR²CO-;
(j) 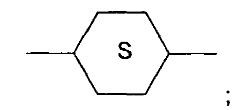 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a nontoxic pharmacologically acceptable acid addition salt, N-oxide, ester or quaternary ammonium salt, or a prodrug thereof; and
b) a pharmaceutically acceptable vehicle, carrier or diluent.

15. A kit as claimed in claim 14 wherein the estrogen agonist / antagonist is a compound of formula (IA): 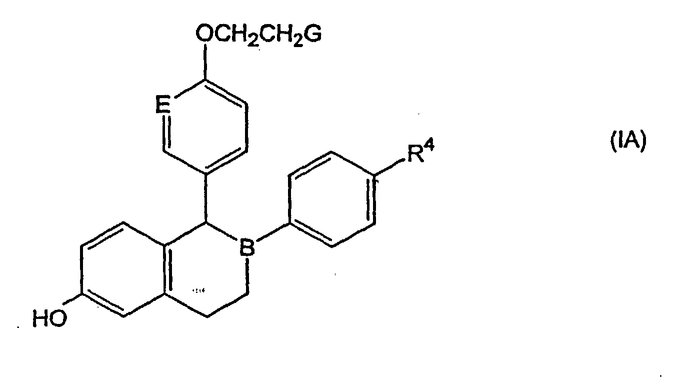 wherein G is 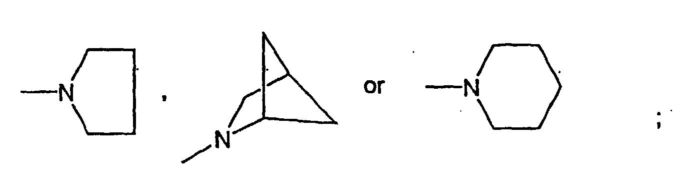 R⁴ is H, OH, F, or Cl; and B and E are independently selected from CH and N or an optical or geometric isomer thereof; or a nontoxic pharmacologically acceptable acid addition salt, N-oxide, ester or quaternary ammonium salt, or a prodrug thereof.

16. A kit as claimed in claim 14 wherein the estrogen agonist / antagonist is a member of the group consisting of
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydro-naphthalene-2-ol;
cis-6-phenyl-5-[4-(2-pyrrolidin-1-yl-ethoxy)-phenyl]-5,6,7,8-tetrahydro-naphthalene-2-ol;
cis-1-[6'-pyrrolidinoethoxy-3'-pyridyl]-2-phenyl-6-hydroxy-1,2,3,4-tetrahydronaphthalene;
1-(4'-pyrrlidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol,
1-(4'-pyrrlidinolethoxyphenyl)-2-phenyl-6-hydroxy-1,2,3,4-tetrahydroisoquinoline and optical or geometric isomers thereof; and nontoxic pharmacologically acceptable acid addition salts, N-oxides, esters and quaternary ammonium salts, and prodrugs thereof.

17. A kit as claimed in claim 14 wherein the compound is cis-6-(4-fluoro phenyl)-5-[4-(2-piperidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

18. A kit as claimed in claim 14 wherein the compound is (-)-cis-6-phenyl-5-[4-(2-pyrrolidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

19. A kit as claimed in claim 14 wherein the compound is cis-6-phenyl-5-[4-2-pyrrolidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

20. A kit as claimed in claim 14 wherein the compound is cis-1-[6'-pyrrolidinoethoxy-3'-pyridyl]-2-phenyl-6-hydroxy-1,2,3,4-tetrahydronaphthalene or an optical or geometric isomer thereof; or a nontoxic pharmacologically acceptable acid addition salt, N-oxide, ester, quaternary ammonium salt, or a prodrug thereof.

21. A kit as claimed in claim 14 wherein the compound is 1-(4'-pyrrlidinoethoxyphenyl)-2-(4"-fluorophenyl)-6-hydroxy-1,2,3,4-tetrahydroisoquinoline or an optical or geometric isomer thereof; or a nontoxic pharmacologically acceptable acid addition salt, N-oxide, ester, quaternary ammonium salt, or a prodrug thereof.

22. A kit as claimed in claim *14* wherein the compound is cis-6-(4-hydroxyphenyl)-5-[4-(2-piperidin-1-yl-ethoxy)-phenyl]-5,6,7,8-tetrahydro-naphthalene-2-ol or an optical or geometric isomer thereof; or a nontoxic pharmacologically acceptable acid addition salt, N-oxide, ester, quaternary ammonium salt, or a prodrug thereof.

23. A kit as claimed in claim 14 wherein the compound is 1-(4'-pyrrlidinolethoxyphenyl)-2-phenyl-6-hydroxy-1,2,3,4-tetrahydroisoquinoline or an optical or geometric isomer thereof; or a nontoxic pharmacologically acceptable acid addition salt, N-oxide, ester, quaternary ammonium salt, or a prodrug thereof.

24. A kit as claimed in 18 wherein the estrogen agonist / antagonist is in the form of a D-tartrate salt.

25. The use of estrogen agonists/antagonists of formula (I): 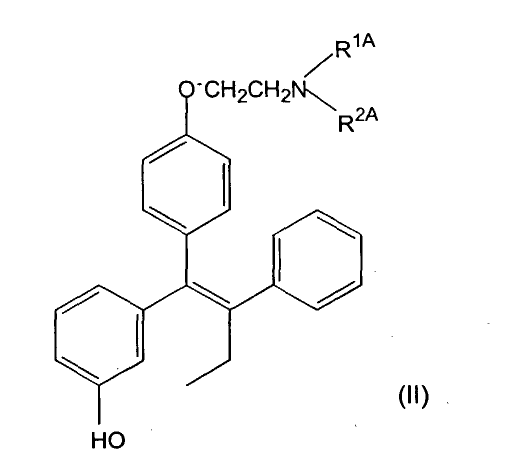 wherein
R^{1A} and R^{2A} may be the same or different provided that, when R^{1A} and R^{2A} are the same, each is a methyl or ethyl group, and, when R^{1A} and R^{2A} are different, one of them is a methyl or ethyl group and the other is hydrogen or a benzyl group; a compound of formulas III or IV : 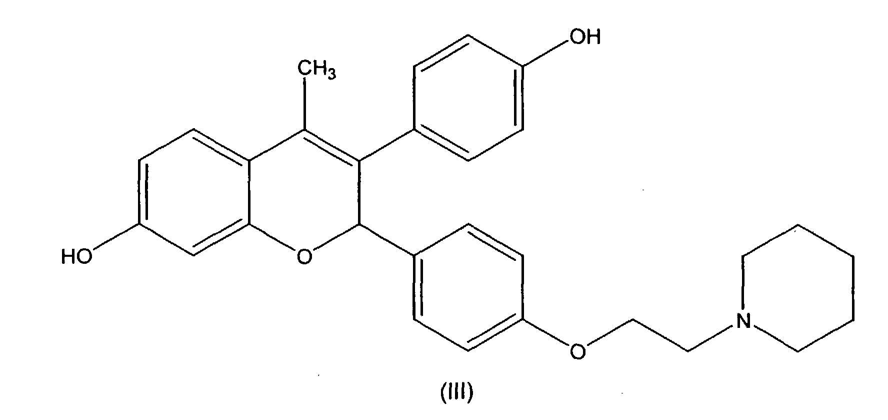 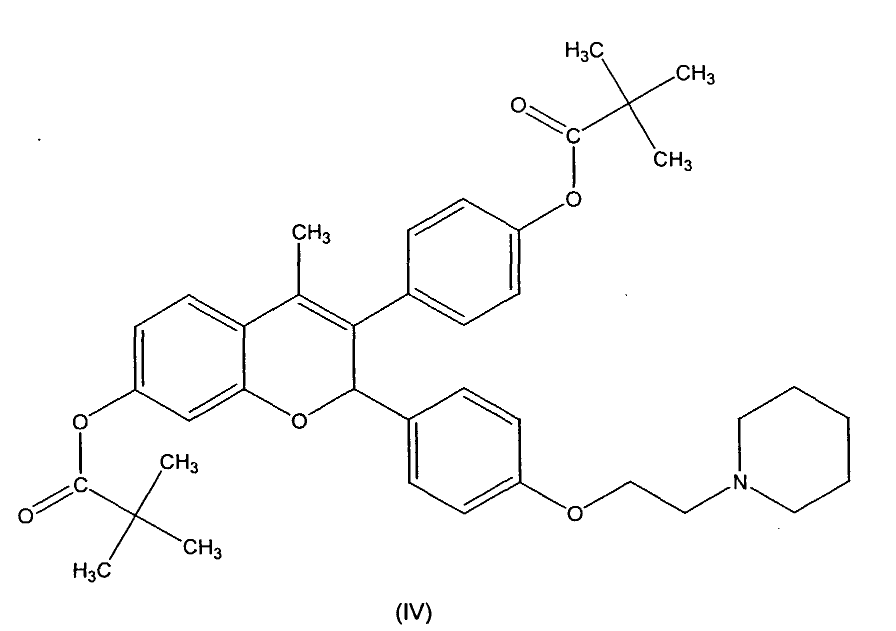 a compound of formulas V and VI: 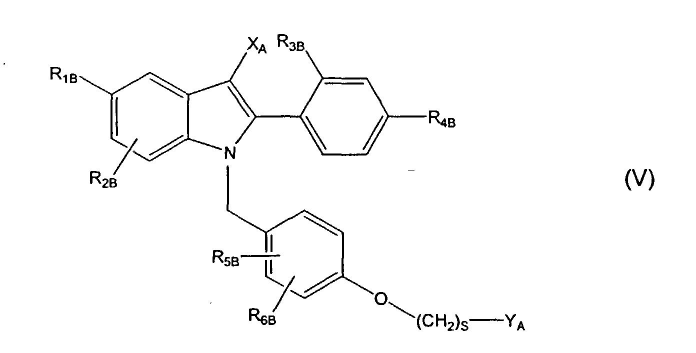 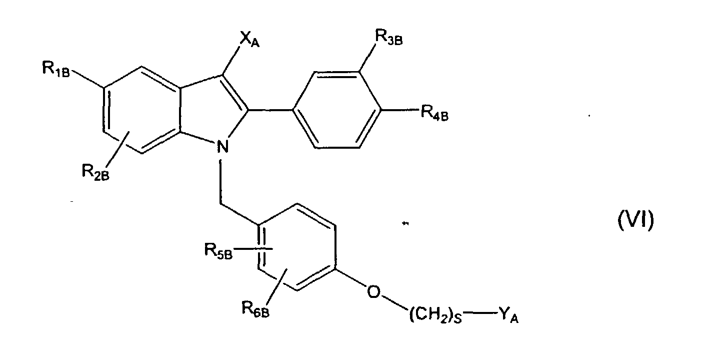 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including trifluoromethyl ether and trichloromethyl ether.
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including trifluoromethyl ether and trichloromethyl ether, cyano, C₁-C₆ alkyl (straight chain or branched), or trifluoromethyl;
X_{A} is selected from H, C₁-C₆ alkyl, cyano, nitro, trifluoromethyl, and halogen;
s is 2 or 3;
Y_{A} is selected from:
a) the moiety: 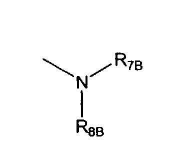 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
b) a five-membered saturated; unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR₁, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
e) a bicyclic heterocycle containing from 6-12 carbon atoms either bridged or fused and containing up to two heteroatoms selected from the group consisting of -O-, -NH-, -N(C₁-C₄ alkyl)-, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H-, -CN-, -CONHR_{1B}-, -NH₂, -N=, C₁-C₄ alkylamino, di(C₁-C₄)alkylamino, -NHSO₂R_{1B}, -NHCOR_{1B}, -NO₂, and phenyl optionally substituted with 1-3 (C₁-C₄) alkyl; or a componud of formula Va: 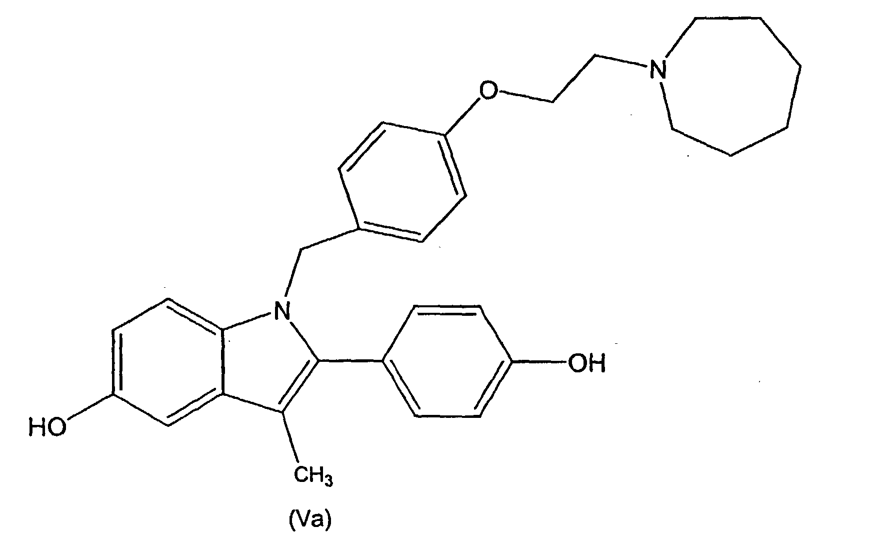 or optical or geometric isomers thereof; nontoxic pharmacologically acceptable acid addition salts, N-oxides, esters, quaternary ammonium salts; or prodrugs thereof, in the manufacture of a medicament to reduce cardiovascular mortality in male and female subjects and reduce morbidity and/or the risk of mortality due to the combined effect of reductions of cardiovascular disease, breast cancer and osteoporosis in post menopausal women.
